# EUROPEAN PATENT SPECIFICATION

(11) **EP 0 633 886 B1**
(45) Date of publication and mention of the grant of the patent: **18.10.2000**
(21) Application number: 93908303.6
(22) Date of filing: 16.03.1993
(51) Int. Cl.: C07D 487/04, C07H 15/26, C07D 519/00, C07D 471/04, C07D 473/16, A61K 31/505

(54) **PHARMACEUTICALLY ACTIVE BICYCLIC-HETEROCYCLIC AMINES**
PHARMAZEUTISCH WIRKSAME BICYCLISCH HETEROCYCLISCHE AMINE
AMINES BICYCLIQUES-HETEROCYCLIQUES EFFICACES PHARMACEUTIQUEMENT

(30) Priority: 03.04.1992 US 863646
(43) Date of publication of application: 18.01.1995
(73) Proprietor: PHARMACIA & UPJOHN COMPANY, Kalamazoo, Michigan 49001 (US)
(72) Inventor: AYER, Donald, E., Kalamazoo, MI 49006 (US); BUNDY, Gordon, L., Portage, MI 49002 (US); JACOBSEN, Eric, Jon, Plainwell, MI 49080 (US)
(74) Representative: Perry, Robert Edward
(86) International application number: US9302188
(87) International publication number: WO9320078

(56) References cited:
- WO-A-91/04254
- GB-A- 864 145
- GB-A- 1 268 772
- JOURNAL OF HETEROCYCLIC CHEMISTRY vol. 25, 1988, PROVO US pages 1633 - 1639 B. VENUGOPALAN ET AL. 'Synthesis of 6,7-dimethoxypyrimido(4,5-b)indoles as potential antihypertensive agents'

## Description

This invention relates to heterocyclic amines, many of which are new, and to their use as pharmaceuticals.

### Background of the Invention

WO 87/01706, WO 87/07895, WO 88/08424 and WO 88/07527 disclose substituted amino type compounds which are useful for treating the same diseases and injuries as those of the present invention.

WO 92/02500 discloses 2-phenylindole derivatives useful for treating asthma, allergic disorders, thrombosis and ischaemia.

J. Heterocyclic Chem. 24:425 (1987) discloses pyrrolopyrimidines substituted at the pyrimidine moiety by amino groups.

WO 91/04254 discloses pyrrolo[2,3-d]pyrimidines where any substituents on the pyrrole ring are simple. In two of the positions the groups are H, halogen or alkyl. In the third, it is H, alkyl or aralkyl.

Venugopalan *et al*, J. Heterocyclic Chem. 25:1633 (1988), discloses 6,7-dimethoxypyrimido[4,5-b]indoles as potential antihypertensive agents. The disclosed compounds include 6,7-dimethoxy-2,4-di(1-piperidinyl)-9H-pyrimido[4,5-b]indole and 6,7-dimethoxy-2,4-di(4-methyl-1-piperazinyl)-9H-pyrimido[4,5-b]indole.

### Summary of the Invention

A first aspect of the present invention is the use defined in claim 1. A second aspect is novel compounds, as defined in claim 2.

### Description of the Invention

Compounds (XXX) of the invention are amines and, as such, form acid addition salts when reacted with acids of sufficient strength. Pharmaceutically acceptable salts include salts of both inorganic and organic acids. The pharmaceutically acceptable salts are preferred over the corresponding free amines since they produce compounds which are more water-soluble and more crystalline. The preferred pharmaceutically acceptable salts include salts of the following acids: hydrochloric, hydrobromic, sulfuric, phosphoric, nitric, citric, methanesulfonic, CH₃-(CH₂)ₙ₁-COOH or HOOC-(CH₂)ₙ₁-COOH where ₙ₁ is 0 to 4, above, HOOC-CH=CH-COOH or ϕ-COOH. For other acceptable salts, see *Int. J. Pharm*., 33, 201-217 (1986). Hydrochloride, hydrobromide, maleate and methanesulfonate salts are most preferred.

It is preferred that -NR₂₋₁R₂₋₂ is the same as -NR₄₋₁R₄₋₂. It is preferred that R₂₋₁ and R₂₋₂ are taken together with the attached nitrogen atom to form 1-pyrrolidinyl, 1-piperazinyl, 1-thiomorpholinyl or 4-methylpiperazin-1-yl; it is more preferred that R₂₋₁ and R₂₋₂ form 1-pyrrolidinyl or 1-piperazinyl. It is preferred that R₇ is -H, -CH₃, -ϕ, 2-(1-morpholinyl)ethyl or 2-(1-piperazinyl)ethyl.

The starting point in the synthesis of the pharmacologically active heterocyclic amines (XXX) is the halogenated pyrimidine ring. Before forming the second ring, the final desired substituents (-NR₂₋₁R₂₋₂ and -NR₄₋₁R₄₋₂) on the heteroaryl ring are added or formed. The substituents -NR₂₋₁R₂₋₂ and -NR₄₋₁R₄₋₂ may be different, but it is preferred that they be the same, for simplicity of chemical synthesis. The formation of the tertiary amines (-NR₂₋₁R₂₋₂, -NR₄₋₁R₄₋₂) from halogenated aromatic/heteroaromatic compounds is known to those skilled in the art, see J. Med. Chem. 33:1145 (1990). Generally, after the desired groups at the C₂ and C₄ positions are formed, the 5-membered ring is formed, by methods known to those skilled in the art. However, in some cases the -NR₂₋₁NR₂₋₂ and NR₄₋₁R₄₋₂ groups are added to the preformed heterocyclic amine (XXX). The -NR₂₋₁NR₂₋₂ and NR₄₋₁R₄₋₂ groups can be cyclized to form rings including 1-pyrrolidinyl, 1-piperidinyl, 1-morpholinyl, 1-piperazinyl, 1-aziridinyl, 1-azetidinyl and a number of other heterocyclic rings. These rings can be substituted with 1 to 3 groups R₂₋₃. When R₂₋₃ is alkyl, no more than two such groups can be on any one carbon atom in the ring. When R₂₋₃ is other than alkyl, only one such group can be on any one carbon atom.

The heterocyclic amines (XXX) are prepared by the process of Chart A and known means, see Comprehensive Heterocyclic Chemistry, A.R. Katritzky and C.W. Rees, Ed., Vol. 4, Pergamon Press, 1984, p. 528. In Chart A, formula VII represents compounds of the invention, since R₅ and R₆ together are -Q-.

The trihalopyrimidines (I) are well known to those skilled in the art or are commercially available. The preferred 2,4,6-trihalopyrimidine (I) is trichloropyrimidine (I). A mixture of the trihalopyrimidine (I) in an inert solvent such as THF is allowed to react with 1 equivalent of a primary amine, R₇-NH₂ (II) in the presence of an acid scavenger. Organic amines such as pyridine, triethylamine, diisopropylethylamine and inorganic bases such as potassium carbonate are useful acid scavengers. The reactants are mixed at a reduced temperature (-80° to 0°) and the reaction mixture is allowed to warm to room temperature (20-25°) and then is often concentrated at reduced pressure. The residue is partitioned between an organic solvent such as ethyl acetate or methylene chloride and an aqueous inorganic base such as potassium bicarbonate. The extract is dried, concentrated, and the residue chromatographed on silica gel to separate the desired 4-aminopyrimidine (III). The 4-aminopyrimidine (III) is mixed with an excess of a secondary amine, NHR₂₋₁R₂₋₂ (IV) and the mixture is heated under reflux for 2 to 24 hours. The mixture is allowed to cool and then is concentrated. The residue is partitioned as described above to remove the inorganic salts. The crude product is purified by conventional means (e.g. crystallization and/or chromatography) to give the desired trisubstituted pyrimidine (V). If a relatively nonvolatile secondary amine is used, the reaction mixture is diluted with an organic solvent such as ethyl acetate and the mixture is washed with an aqueous inorganic base. Alternatively, the required trisubstituted pyrimidine (V) intermediate may be obtained from reaction of a 2,4-di-amino-6-halopyzidine with the appropriate primary amine (II) at elevated temperatures. The trisubstituted pyrimidine (V) is contacted with an α-haloketone, R₅-CHX₁-CO-R₆ (VI) where X₁ is preferably -Cl or -Br which provides a ketopyrimidine intermediate. The ketopyrimidine may cyclize to the desired pyrrolo[2,3-d]pyrimidine (VII) spontaneously at 20-25°. The cyclization may be accomplished by warming the ketopyrimidine intermediate in an inert solvent (e.g. THF, ethyl acetate, toluene, methylene chloride) in the presence (or absence) of a mild dehydrating agent such as magnesium sulfate, molecular sieve, trialkyl orthoformate, etc. The cyclization may also be achieved by chromatography of the intermediate on silica gel in the conventional way. The final product is purified by chromatography and/or crystallization.

The heterocyclic amines (XXX) are useful in treating/preventing spinal trauma, mild and/or moderate to severe head injury, subarachnoid hemorrhage and subsequent ischemic (thromboembolic) stroke, asthma and reduction of mucous formation/secretion in the lung, muscular dystrophy, adriamycin cardiac toxicity, Parkinsonism, Alzheimer's disease, other degenerative neurological disorders, multiple sclerosis, organ damage during reperfusion after transplant, skin graft rejection, hemorrhagic, traumatic and septic shock, and conditions such as severe bums, ARDS, inflammatory diseases such as osteo- or rheumatoid arthritis, nephrotic syndrome (immunological), systemic lupus erythematosis, allergic reactions, diabetes, atherosclerosis, inflammation (dermatological antiinflammatory and antipsoriasis agents), emphysema, cancer (limit metastasis, limit tumor growth), (stress-induced) ulcers, ulcerative colitis and Crohn's disease. The compounds are also useful for prophylactic treatment before neurological procedures, for treatment of myocardial infarctions, drug allergic reactions, post-resuscitation ischemia, end migraine headaches. The compounds have use in ophthalmology, e.g., in treatment of diabetic retinopathy, age-related macular degeneration, cataracts and glaucoma, light-induced retinal damage and in irrigation mixtures used in eye surgery.

In humans, the heterocyclic amines (XXX) of the present invention are useful in treating subarachnoid hemorrhage and subsequent cerebral vasospasm, global cerebral ischemia, with resuscitation (CPR) to prevent post-ischemic brain damage, brain tumor (neuroprotective), Bells Palsy, other degenerative neurological disorders, hepatic necrosis (e.g. from viral hepatitis), some forms of radiation damage (for example during radiation treatment or from accidental exposure to radiation), myocardial damage after myocardial ischemia, pre-birth infant strangulation and infant hypoxia syndrome, such opthalmic disorders as uveitis and optic neuritis and ischemic bowel syndrome.

In humans, the heterocyclic amines (XXX) are useful in preventing damage following cardiopulmonary resuscitation, neurological or cardiovascular surgery and from cardiac infarction, ocular damage after opthalmic surgery (e.g. cataritic surgery).

It is preferred that the heterocyclic amines (XXX) are useful in treating asthma (and reduction of mucous formaation/secretion in the lung), muscular dystrophy, Parkinsonism, Alzheimer's disease and stroke.

Generally, the heterocyclic amines (XXX) are used like the glucocorticoid pharmaceuticals for the treatment of the above human conditions as well as the animal conditions listed below. While the heterocyclic amines (XXX) are useful in both humans and animals in treating many of the same conditions and preventing damage from the same problems as the glucocorticoids, the heterocyclic amines (XXX) are useful in treating a number of conditions and preventing damage from conditions where the glucocorticoids are not useful. The heterocyclic amines (XXX) have no glucocorticoid activity and therefore, unlike the glucocorticoids, they can be given daily for long periods of time (used chronically) without the side effects associated with the glucocorticoids. This is a distinct advantage.

It is to be understood that each of the heterocyclic amines (XXX) is useful to a different degree for treating each of the conditions above. However, as is known to those skilled in the art, some of the bicyclic heterocyclic amines (XXX) are better for treating some conditions and others are better for treating other conditions.

The rat brain malonyldialdehyde and mouse spinal neuron lipid peroxidation assays (Hall et al, J. of Pcol. Exptl. Ther., 258, 688-694 (1991) identifies compounds which are antioxidants, which inhibit lipid peroxidation, and are useful in treating spinal trauma, mild and/or moderate to severe head injury, degenerative neurological disorders, etc. This test also will permit one skilled in the art to determine the relative degree to which each of the heterocyclic amines (XXX) are useful and which are the preferred compounds. Another method useful for determining which particular compounds inhibit lipid peroxidation, and which are therefore useful in treating spinal trauma, mild and/or moderate to severe head injury, degenerative neurological disorders, etc is described by Pryor in Methods of Enzymology 105, 293 (1984). This test also will permit one skilled in the art to determine the relative degree to which each of the heterocyclic amines (XXX) are useful and which are the preferred compounds. Further, the mouse head injury assay of Hall, J. Neurosurg., 62, 882 (1980) discloses an assay from which one skilled in the art can readily determine which particular heterocyclic amines (XXX) are useful in the acute treatment of spinal trauma or mild and/or moderate to severe head injury. This test also will permit one skilled in the art to determine the relative degree to which each of the heterocyclic amines (XXX) are useful and which are the preferred compounds. Additionally, the cat 48 hour motor nerve degeneration model of Hall et at Exp. Neurol., 79, 488 (1983) discloses a routine assay from which one skilled in the art can readily determine which particular heterocyclic amines (XXX) are useful in treating chronic degenerative neurological disorders such as Parkinsonism, Alzheimer's disease etc. This test also will permit one skilled in the art to determine the relative degree to which each of the heterocyclic amines (XXX) are useful and which are the preferred compounds. H. Johnson in Int. Arch. Allergy Appl. Immunol., 70, 169 (1983) has described the ascaris sensitized rhesus monkey assay for anti-asthma drugs.

The standard conditions for treatment are to give the heterocyclic amines (XXX) orally or parenterally, e.g. IV (that is by injection, infusion or continuous drip) or IM, with a standard dose of about 0.05 to about 20 mg/kg/day IV for up to 10 days or about 0.05 to about 20 mg/kg/day; one to four times daily by mouth.

For treating spinal trauma, mild and moderate to severe head injury, damage following cardiopulmonary resuscitation, cardiac infarction, organ damage during reperfusion after transplant, hemorrhagic, traumatic and septic shock, severe burns, ARDS, and nephrotic syndrome and preventing skin graft rejection, the standard conditions are used. Typical treatment will involve an initial loading dose, e.g. an IV dose of 0.01 mg to 2 mg/kg followed by maintenance dosing e.g. IV infusion for a day to a week depending on the particular condition of the patient and the particular compound used. This may be supplemented with IM or oral dosing for days, weeks or months to prevent delayed neuronal degeneration in neurological applications (eg spinal trauma, head injury).

In treating subarachnoid hemorrhage and subsequent cerebral vasospasm or ischemic (thromboembolic) stroke the standard conditions are used and patients at risk are pre-treated orally.

In treating excess mucous secretion and asthma, the heterocyclic amines (XXX) are administered orally, IV and by inhalation in the standard dose. In treating excess mucous secretions, the oral dose of the heterocyclic amines (XXX) used is from about 0.05 to about 20 mg/kg/day. The frequency of administration is one thru 4 times daily. The oral administration of the heterocyclic amines (XXX) to treat excess mucous secretions may go on for months or even years. The susceptible individuals can be pre-treated a few hours before an expected problem. The IV dose is about 0.05 to about 20 mg/kg/day. The aerosol formulation contains about 0.01 to about 1.0% of the bicyclic heterocyclic amines (XXX) and is administered or used about four times daily as needed.

In treating muscular dystrophy, Parkinsonism, Alzheimer's disease and other degenerative neurological disorders (amyotrophic lateral sclerosis; multiple sclerosis), heterocyclic amines (XXX) are administered orally using a dose of about 0.05 to about 20 mg/kg/day, administered or used one to four times a day. The treatment may go on for years.

In addition, utility in disorders or physiological phenomena dependent on angiogenesis or neovascularization such as embryo implantation (antifertility), arthritis, and atherosclerosis is exhibited with the heterocyclic amines (XXX) with or without co-administered oral heparin or systemic heparin fragments, see Science 221, 719 (1983).

In treating adriamycin-induced cardiac toxicity, the heterocyclic amines (XXX) are administered orally or IV using a dose of about 0.05 to about 50 mg/kg/day, preferrably about 0.5 to about 10 mg/kg/day. The heterocyclic amines (XXX) are preferably given concomitantly with IV adriamycin or the individual is pre-treated with the heterocyclic amines (XXX).

For prophylaxis prior to and preventing damage after neurological or cardiovascular surgery, the heterocyclic amines (XXX) are used according to the standard conditions. The patient can be pretreated with a single IV or IM dose just prior to surgery or orally before and after surgery.

In treating osteo- or rheumatoid arthritis and other inflammatory diseases, the heterocyclic amines (XXX) are given orally or IM in doses of about 0.05 to about 20 mg/kg/day, one to four times daily. Orally the drug will be given over a period of months or years alone or with other steroidal or nonsteroidal antiinflammatory agents. The initial dose with some severe rheumatoid patients may be given IV and followed with an IV drip for up to 24 hours or more. In addition, intra-arterial administration may be employed.

In treating drug allergic reactions, the heterocyclic amines (XXX) are given in a dose of about 0.05 to 20 mg/kg/day, administered one to four times daily orally and IV. Typical treatment would be an initial IV loading dose followed by oral dosing for a few days or more.

In treating atherosclerosis and emphysema, the heterocyclic amines (XXX) are given orally in a dose of about 0.05 to about 20 mg/kg/day, one to four times daily for months or years.

In treating dermatological inflammatory conditions including psoriasis, the heterocyclic amines (XXX) are given orally in a dose of about 0.05 to about 20 mg/kg/day, one to four times daily or applied topically as a cream, ointment or lotion or equivalent dosage form in a concentration of about 0.05 to about 5% as long as needed. In treating these conditions the heterocyclic amines (XXX) can be used with steroidal agents.

The heterocyclic amines (XXX) are useful in the prevention and treatment of stress ulcers and of gastric intolerance caused by drugs such as nonsteroidal anti-inflammatory compounds (NOSAC). Stress ulcers are ulcers that develop after exposure to severe conditions such as trauma, bums, sepsis, extensive surgery, acute illnesses, and the like. Patients in intensive care units are particularly prone to develop stress ulcers. Stress ulcers also include lesions that can lead to upper gastrointestinal bleeding; such bleeding is likely to be prevented by these compounds. NOSAC includes drugs such as ibuprofen, aspirin, indomethacin, naproxen, piroxicam and the like that are usually taken for analgesia, and that are often associated with gastrointestinal intolerance characterized by pain and lesions that may lead to bleeding. The heterocyclic amines (XXX) will be administered preferentially by the oral mute either as tablets, capsules or liquids, in doses ranging from about 5 to about 500 mg, two to four times a day. The treatment would be either preventive, i.e., starting before ulcers have formed in patients at risk of developing such lesions, or therapeutic, i.e., once the ulcers have formed. In patients whose clinical condition precludes swallowing the oral dosage forms, the heterocyclic amines (XXX) would be given either through a nasogastric tube, or parenterally, i.e., IV or IM. The parenteral doses would range from about 1 to about 100 mg and be administered one to four times a day or by IV.

In dogs, the heterocyclic amines (XXX) are useful in treating trauma, intervertebral diseases (slipped disk), traumatic shock, flea bite and other allergies.

In horses, the heterocyclic amines (XXX) are useful in treating endotoxic or septic shock which follows colic, pretreatment before surgery for colic and treatment of Founder (laminitis).

In cattle, the heterocyclic amines (XXX) are useful in treating acute coliform mastitis, bovine mastitis, acute allergic reaction to feed lot vaccination and shipping fever.

In pigs, the heterocyclic amines (XXX) are useful in treating porcine stress syndrome and thermal stress syndrome.

The term treatment or treating as used in this patent is used broadly and includes both treatment of an existing condition as well as preventing the same condition from occurring where such is possible as is well known to those skilled in the art. For example, the heterocyclic amines (XXX) can be used to treat existing asthma conditions and to prevent future ones from occurring. For example, the heterocyclic amines (XXX) treat spinal trauma and prevent rejection of skin grafts.

The heterocyclic amines (XXX) can be used with other pharmaceutical agents in treatment of the conditions listed above as is known to those skilled in the art.

The exact dosage and frequency of administration depends on the particular heterocyclic amines (XXX) used, the particular condition being treated, the severity of the condition being treated, the age, weight, general physical condition of the particular patient, other medication the individual may be taking as is well known to those skilled in the art and can be more accurately determined by measuring the blood level or concentration of the heterocyclic amines (XXX) in the patient's blood and/or the patient's response to the particular condition being treated.

### DEFINITIONS AND CONVENTIONS

The definitions and explanations below are for the terms as used throughout this entire document including both the specification and the claims.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-C(=Z₁)H. Groups Rᵢ and Rⱼ would represent monovalent variable substituents if attached to the formula CH₃-CH₂-C(Rᵢ)(Rⱼ)H₂. When chemical formulas are drawn in a liner fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom. Also, for any molecule with an established system of carbon atom numbering, such as steroids, these carbon atoms are designated as Cᵢ, where "i" is the integer corresponding to the carbon atom number. For example, C₆ represents the 6 position or carbon atom number in the steroid nucleus as traditionally designated by those skilled in the art of steroid chemistry. Likewise the term "R₇" represents a variable substituent (either monovalent or bivalent) at the C₇ position.

Chemical formulas or portions thereof drawn in a linear fashion represent atoms in a linear chain. The symbol "-" in general represents a bond between two atoms in the chain. Thus CH₃-0-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)-CH=CCl-CH=C*H with the convention that the atoms marked with an asterisk (∗) are bonded to each other resulting in the formation of a ring. Likewise, the cyclic molecular fragment, 4-(ethyl)-1-piperazinyl can be represented by -N*-(CH₂)₂-N(C₂H₅)-CH₂-C*H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In chemical structural formulas depicting such compounds, a substituent (X₁) which is "below" another substituent (X₂) will be identified as being in the alpha (α) configuration and is identified by a broken, dashed or dotted line attachment to the carbon atom, i.e., by the symbol "- - -" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, the two monovalent variable substituents are α-R₆₋₁:β-R₆₋₂, .... α-R₆₋₉:β-R₆₋₁₀, etc, giving -C(α-R₆₋₁)(β-R₆₋₂)-, .... -C(α-R₆₋₉)(β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CO-O-CH₂-CH₂-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-0-CO- where n is zero, one or two. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

When the claims contain a fairly complex (cyclic) substituent, at the end of the phrase naming/designating that particular substituent will be a notation in (parentheses) which will correspond to the same name/designation in one of the CHARTS which will also set forth the chemical structural formula of that particular substituent.

### II. DEFINITIONS

All temperatures are in degrees Centigrade.
TLC refers to thin-layer chromatography.
THF refers to tetrahydrofuran.
DMF refers to dimethylformamide.
Saline refers to an aqueous saturated sodium chloride mixture.
IR refers to infrared spectroscopy.
CMR refers to C-13 magnetic resonance spectroscopy, chemical shifts are reported in ppm (δ) downfield from tetramethylsilane.
NMR refers to nuclear (proton) magnetic resonance spectroscopy, chemical shifts are reported in ppm (δ) downfield from tetramethylsilane.
TMS refers to tetramethylsilane.
-φ refers to phenyl (C₆H₅).
MS refers to mass spectrometry expressed as m/z or mass/charge unit. [M + H]⁺ refers to the positive ion of a parent plus a hydrogen atom. EI refers to electron impact. CI refers to chemical ionization. FAB refers to fast atom bombardment.
HRMS refers to high remixture mass spectrometry.
Ether refers to diethyl ether.
Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.
When solvent pairs are used, the ratios of solvents used are volume/volume (v/v).
When the solubility of a solid in a solvent is given the ratio of the solid to the solvent is weight/volume (w/v).

### EXAMPLES

The following Examples illustrate the preparation of compounds of the invention. In particular, the compounds of Examples 8 to 12 are preferred compounds of the invention. The compound of Example 8 is most preferred. Examples A, B, C and D illustrate in detail procedures that are used in other Examples.

### Example 1 2,6-Dichloro-4-methylaminopyrimidine (III)

2,4,6-Trichloropyrimidine (I, 30 g) is added to a suspension of methylamine hydrochloride (II, 10 g) in THF (250 ml). The mixture is cooled to -6° and diisopropylethylamine (55 ml) is added slowly. The mixture is stirred for 3 days at 20-25° and then is concentrated under reduced pressure. The residue is absorbed on silica gel (75 g) with ethyl acetate/methylene chloride (1/1) and applied to a silica gel (1 kg) column packed in ethyl acetate/hexane (1/1). Elution is performed with ethyl acetate/hexane (1/1) collecting 500 ml fractions. The appropriate fractions (8-14) are pooled and concentrated to give the title compound, NMR (CDCl₃) 6.29, 6.2 and 2.9 δ.

### Example 2 4-Methylamino-2,6-di(1-pyrrolidinyl0pyrimidine (V)

Pyrrolidine (IV, 25 ml) is added (exothermic) to 2,6-dichloro-4-methylaminopyrimidine (III, Example 1, 1.81 g). The mixture is stirred and heated under reflux for 23 hours, then is allowed to cool and concentrated under reduced pressure. The residue is partitioned between ethyl acetate and aqueous potassium bicarbonate, the phases separated and organic phase is concentrated to give a solid. The solid is crystallized from hexane to give the title compound, mp 100.5-103°; NMR (CDCl₃) 4.74, 3.51, 3.43, 2.81 and 1.9 δ; CMR (CDCl₃) 164.50, 161.92, 160.18, 70.78, 46.06, 45.85, 28.47, 25.44, 25.19 δ.

Alternatively, the title compound can be obtained by the reaction of 4-chloro-2,6-di(1-pyrrolidinyl)pyrimidine and methylamine (II) in pyridine in a pressure tube at 100°, MS (M+) 247.

### Example 3 2-[(2,6-Dichloropyrimidin-4-yl)amino]ethanol (III)

Following the general procedure of Example 1 and making non-critical variations but starting with ethanolamine (II, 1.65 ml) and 2,4,6-trichloropyrimidine (I, 5.00 g), the title compound is obtained, NMR (CDCl₃) 6.33, 3.86, 3.59, 1.59 δ.

### Example 4 2-[(2,6-Di(1-pyrrolidinyl)pyrimidin-4-yl)amino]ethanol (V)

Following the general procedure of Example 2 and making non-critical variations but starting with pyrrolidine (IV, 10.0 ml) and 2-[(2,6-dichloropyrimidin-4-yl)amino]ethanol (III, Example 3, 2.77 g), the title compound is obtained, mp 138-140°; IR (mineral oil) 1600, 1571, 1508, 1476, 1449, 1432, 1417, 1343 cm⁻¹; NMR (CDCl₃) 5.9-6.6, 4.8-5.1, 4.76, 3.74, 3.25-3.6, 1.7-2.0 δ; MS (m/z) 277, 249, 233 and 205.

### Example 5 4-tert-Butylamino-2,6-dichloropyrimidine (III)

Tert-butylamine (II, 6.23 ml) is slowly added to 2,4,6-trichloropyrimidine (I, 10.0 g) at -20° (temp. rose to -14°). Diisopropylethylamine (9.50 ml) is added and the mixture is stirred at 20-25° for 72 hours. Basic workup (ethyl acetate, 1 N potassium bicarbonate, magnesium sulfate) and purification by flash chromatography eluting with hexane/ethyl acetate (9/1), pooling and concentrating the desired fractions gives the title compound, mp 192-193°; NMR (CDCl₃) 6.29, 1.44 δ.

### Example 6 4-tert-Butylamino-2,6-di(1-pyrrolidinyl)pyrimidine (V)

Pyrrolidine (IV, 10.0 ml) is added to 4-tert-butylamino-2,6-dichloropyrimidine (III, Example 5, 3.55 g) at -10°, and the mixture heated at reflux for 30 hours. After cooling to 20-25°, basic workup (ethyl acetate, 1 N potassium bicarbonate, saline wash, magnesium sulfate) gives the title compound as a liquid; NMR (CDCl₃) 4.78, 4.26, 3.3-3.6, 1.85-2.0 and 1.41 δ.

### Example 7 2-Bromocyclohexanone (VI)

A mixture of cyclohexanone (3.25 ml) in 30 ml ethyl acetate/chloroform (1/1) is prepared in a 100 ml flask. To this stirring mixture, copper bromide (13.96 g) is added as a solid in one portion. The contents of the flask were brought to reflux for 1.25 hours. The reaction is then cooled to 20-25° followed by filtration. The filtrate is washed with 3 x 20 ml portions of saturated sodium bicarbonate mixture. The organic layer is dried over sodium sulfate, filtered, and the solvent is removed under reduced pressure. An oil (4.29 g) is obtained which is chromatographed on 455 g of silica gel. The eluent (3.5 l) is ethyl acetate/hexane (19/1) followed by (9/1, 1.5 l). Like fractions were combined based on TLC homogeneity and concentrated to give the title compound, NMR (CDCl₃, TMS) 4.45, 3.04-2.94, 2.39-2.19, 2.05-1.94, 1.87-1.71 δ.

### Example 8 5,6,7,8-Tetrahydro-9-methyl-2,4-di(1-pyrrolidinyl)-5H-pyrimido[4,5-b]indole (VII)

A mixture of 4-methylamino-2,6-di(1-pyrrolidinyl)pyrimidine (V, Example 2, 1.54 g), diisopropylethylamine (1.15 ml) and 80 ml acetonitrile is prepared in a 200 ml flask. 2-Bromocyclohexanone (VI, Example 7, 1.11 g) is added to the stirring mixture at 20-25°. The reaction is heated at reflux for 29 hr. The contents of the flask were cooled to 0°. A solid precipitated, is collected by filtration and dried to give the title compound, mp 196-200°; NMR (CDCl₃,TMS) 3.68, 3.59, 3.51, 2.72, 2.61, 1.94-1.77 δ.

### Example 9 5,6,7,8-Tetrahydro-9-methyl-2,4-di(1-pyrrolidinyl)-5H-pyrimido[4,5-b]indole, monomethanesulfonate (VII-salt)

Following the general procedure of Example D and making non-critical variations but starting with 5,6,7,8-tetrahydro-9-methyl-2,4-di(1-pyrrolidinyl)-5H-pyrimido[4,5-b]indole (VII, Example 8), the title compound is obtained, mp 178-182°; NMR (CDCl₃,TMS) 3.81-3.73, 2.78, 2.67, 2.57, 2.01-1.76 δ; HRMS (EI, m/z) M⁺ observed = 325.2273, calc'd for C₁₉H₂₇N₅ = 325.2266, other ions observed at m/z 297, 270, 256, 162, 141. IR (mineral oil) 2269, 1625, 1569, 1442, 1247, 1164, 1036, 766 cm⁻¹.

### Example 10 9-Methyl-2,4-di(1-pyrrolidinyl)-9H-pyrimido[4,5-b]indole

A suspension of 5,6,7,8-tetrahydro-9-methyl-2,4-di(1-pyrrolidinyl)-5H-pyrimido[4,5-b]indole (VII, Example 8, 1.86 g) and palladium-on-carbon (10%, 1.52 g) in decalin (225 ml) is heated at reflux for 45 min. After cooling, chromatographic purification [silica gel, hexane, then acetone/methylene chloride (2/98)] followed by recrystallization (methylene chloride/hexane) gives the title compound, mp 153-154°; NMR (CDCl₃) 7.88, 7.23, 7.10, 3.92, 3.75, 3.65 and 1.95; HRMS (EI) M⁺ observed at m/z 321.1957, calc'd for C₁₉H₂₃N₅ = 321.1953.

### Example 11 5,6,7,8-Tetrahydro-2,4-di(1-pyrrolidinyl)1H-pyrimido[4,5-b]indole (VII)

Following the general procedure of Example 8 but starting with 4-tert-butylamino-2,6-di(1-pyrrolidinyl)pyrimidine (V, Example 6) instead of the Example 2 product, the 9-t-butyl derivative of the title compound is obtained. Removal of the t-butyl group is done according to the procedure of Example A.

Following the general procedure of Example B, the methanesulfonate salt of the title compound is obtained, mp 253-254°; NMR(CDCl₃; TMS) 12.47, 11.99, 3.74, 3.60, 2.89, 2.67, 1.93, 1.78 δ; MS (free base) calc'd for C₁₈H₂₅N₅ = 311.2110, found = 311.2109.

### Example 12 5,6,7,8-Tetrahydro-9-[2-(1-piperazinyl)ethyl]-2,4-di(1-pyrrolidinyl)-5H-pyrimido[4,5-b]indole maleate (VII)

Following the general procedure of Example 8 and making non-critical variations, but starting with 2-[(2,6-di(1-pyrrolidinyl)pyrimidin-4-yl)amino]ethanol (V, Example 4), the N-9 hydroxyethyl intermediate corresponding to the title compound is obtained. Conversion of this intermediate to the title compound is accomplished using the general procedures of Examples C and D, making non-critical variations, mp 155-157°; MS (free base) calc'd for C₂₄H₃₇N₇ = 423.3110, found = 423.3113.

### Example A

Trifluoroacetic acid (18.0 ml) is slowly added to a mixture of 7-tert-butyl-6-phenyl-2,4-di(1-pyrrolidinyl)-7H-pyrrolo[2,3-d]pyrimidine(1.2 g) and methylene chloride (4.0 ml) at 0°. The mixture is permitted to warm to 20-25° and the resulting mixture is stirred at 20-25° for 4 hr. Concentration and basic workup (chloroform, 1 N sodium hydroxide, sodium sulfate) gives 6-phenyl-2,4-di(1-pyrrolidinyl)-7H-pyrrolo[2,3-d]pyrimidine.

### Example B

A suspension of 6-phenyl-2,4-di(1-pyrrolidinyl)-7-methyl-7H-pyrrolo[2,3-d]pyrimidine (500 mg) in 50 ml of 2-propanol/water (95/5) is treated with 4.7 ml of a 0.308 M methanesulfonic acid mixture in 2-propanol/water (95/5), and the reaction mixture is stirred at 20-25° for 1 hour. The reaction mixture became homogenous within 1 hr. The reaction mixture is filtered and then concentrated under reduced pressure. The crude product is triturated with ethyl acetate/hexane (1:1, 70 ml) at 5° for 30 min in the dark. The solid is isolated in the dark and dried in a vacuum oven (24 hr, 0.005 mm, 40°) to give 7-methyl-6-phenyl-2,4-di(1-pyrrolidinyl)-7H-pyrrolo[2,3-d]pyrimidine, methanesulfonate.

### Example C

Methanesulfonyl chloride (0.25 ml) is added to a mixture of 2-[6-phenyl-2,4-di(1-pyrrolidinyl)-7H-pyrrolo[2,3-d]pyrimidin-7-yl]ethanol (0.83 g), triethylamine (1.0 ml) and THF (20 ml) at 0°. The mixture is stirred at 0° for 1 hour, quenched with ice, and concentrated. Aqueous workup (ethyl acetate, saline wash, magnesium sulfate) gives 2-[6-phenyl-2,4-di(1-pyrrolidinyl)-7H-pyrrolo[2,3-d]pyrimidin-7-yl]ethylmethanesulfonate.

### Example D

A mixture of piperazine (10.3 g) in acetonitrile (25 ml) is added to a mixture of 2-[6-methyl-2,4-di(1-pyrrolidinyl)-7H-pyrrolo[2,3-d]pyrimidin-7-yl]ethyl methanesulfonate (0.875 g), sodium iodide (10.0 mg), potassium carbonate (1.18 g) and acetonitrile (25 ml) at 20-25°. The mixture is heated at reflux for 7 hr. After cooling to 20-25°, basic workup (1 N potassium carbonate, methylene chloride, sodium sulfate) and purification by flash chromatography eluting with methanol/methylene chloride (5/95), pooling and concentrating the desired fractions gives 6-methyl-7-[2-(1-piperazinyl)ethyl)]-2,4-di(1-pyrrolidinyl)-7H-pyrrolo[2,3-d]pyrimidine.

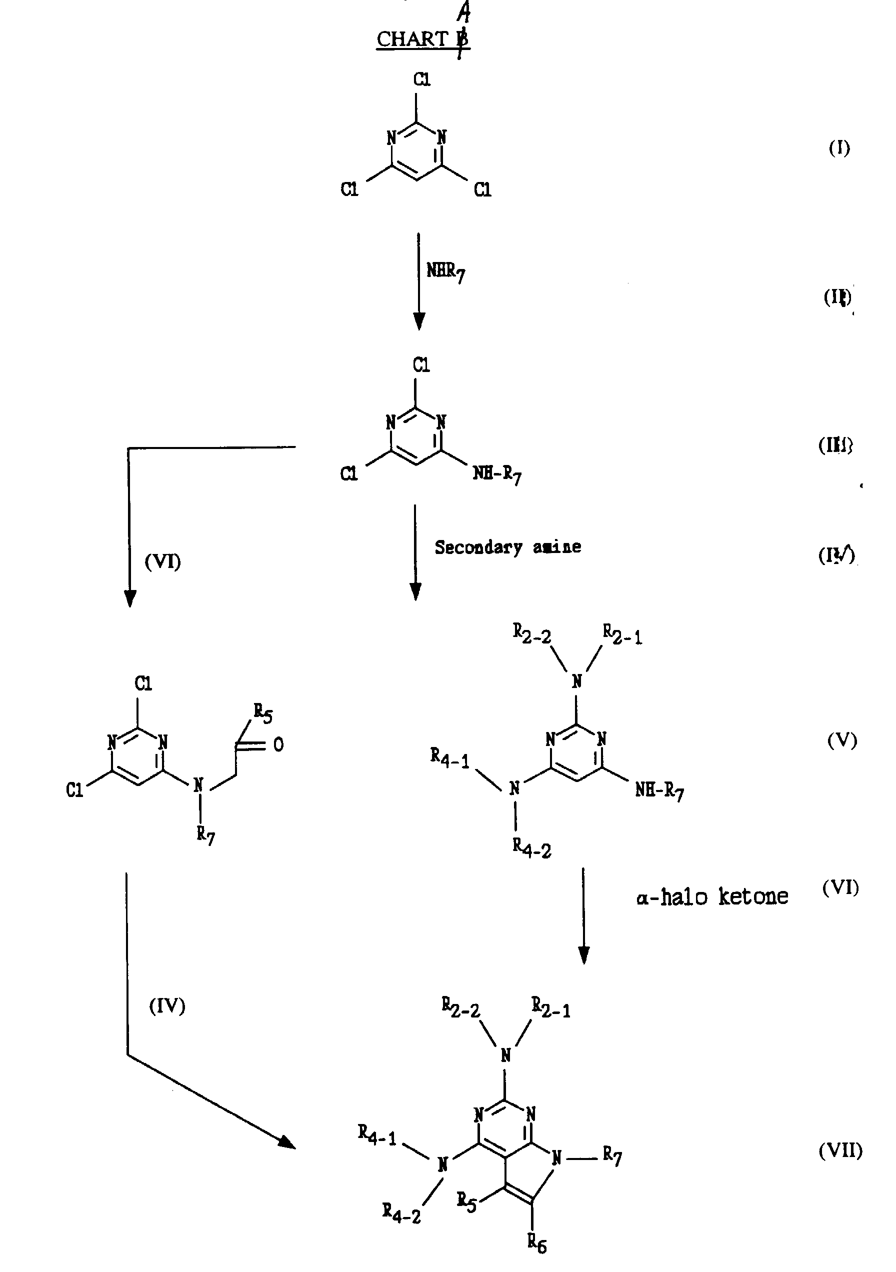

## Claims

1. Use of a compound for the manufacture of a medicament for use in treating/preventing spinal trauma, mild and/or moderate to severe head injury, subarachnoid haemorrhage and subsequent ischemic (thromboembolic) stroke, asthma and reduction of mucous formation/secretion in the lung, muscular dystrophy, adriamycin cardiac toxicity, Parkinsonism, Alzheimer's disease, other degenerative neurological disorders, multiple sclerosis, organ damage during reperfusion after transplant, skin graft rejection, haemorrhagic, traumatic and septic shock, and conditions such as severe bums, ARDS, inflammatory diseases such as osteo- or rheumatoid arthritis, nephrotic syndrome (immunological), systemic lupus erythematosis, allergic reactions, diabetes, atherosclerosis, inflammation (dermatological anti-inflammatory and anti-psoriasis agents), emphysema, cancer (limit metastasis, limit tumour growth), (stress-induced) ulcers, ulcerative colitis, Crohn's disease, myocardial infarctions, drug allergic reactions, post-resuscitation ischemia, or migraine headaches, or in ophthalmology, e.g. in treatment of diabetic retinopathy, age-related macular degeneration, cataracts and glaucoma, light-induced retinal damage and in irrigation mixtures used in eye surgery, in treating subarachnoid haemorrhage and subsequent cerebral vasospasm, global cerebral ischemia, and in preventing post-ischemic brain damage, brain tumour (neuroprotective), Bells Palsy, other degenerative neurological disorders, hepatic necrosis (e.g. from viral hepatitis), radiation damage (for example during radiation treatment or from accidental exposure to radiation), myocardial damage after myocardial isehemia, pre-birth infant strangulation and infant hypoxia syndrome, such ophthalmic disorders as uveitis and optic neuritis and ischemic bowel syndrome, damage following cardiopulmonary resuscitation, neurological or cardiovascular surgery and from cardiac infarction, or ocular damage after ophthalmic surgery (e.g. cataract surgery), wherein the compound is a tricyclic heterocyclic amine of the formula 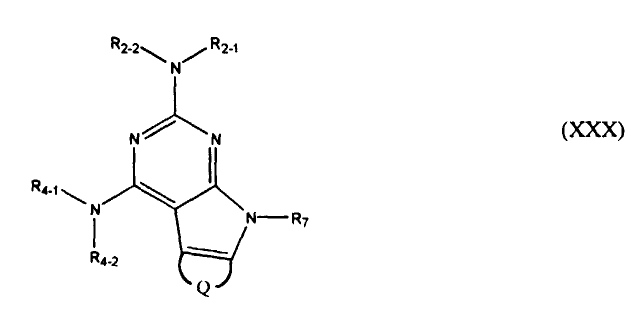
where Q is -(CH₂)₃₋₅- or -CR₅₆₋₁=CR₅₆₋₂-CR₅₆₋₃=CR₅₆₋₄, where R₅₆₋₁, R₅₆₋₂, R₅₆₋₃ and R₅₆₋₄ are -H, -F, -Cl, -Br, -OH, -OCH₃, -OCH₂-φ, -NO₂, C₁-C₃ alkyl, -NH₂, -NHCH₃, N(CH₃)₂ or -CO₂R₅₆₋₅ where R₅₆₋₅ is -H, C₁-C₄ alkyl, C₆-C₁₂ aryl or C₆-C₁₂ aralkyl;
where R₂₋₁ is
(A) -H, or
(B) C₁-C₈ alkyl optionally substituted with 1 to 4
(1) -F,
(2) -Cl,
(3) -OR₂₋₁₀ where R₂₋₁₀ is
(a) -H,
(b) C₁-C₄ alkyl,
(c) phosphate,
(d) sulfate,
(e) -CO-R₂₋₁₁ where R₂₋₁₁ is C₁-C₄ alkyl or C₆-C₉ aralkyl,
(f) -CO-NR₂₋₁₂R₂₋₁₃ where R₂₋₁₂ and R₂₋₁₃ are the same or different and are -H or C₁-C₃ alkyl,
(g) sulfamate,
(h) glucosyl,
(i) galactosyl,
(j) glucuronic acid,
(k) maltosyl,
(l) arabinosyl,
(m) xylosyl,
(n) -CO-CH(NH₂)-H,
(o) -CO-CH(NH₂)-CH₃,
(p) -CO-CH(NH₂)-CH(CH₃)₂,
(q) -CO-CH(NH₂)-CH₂-CH(CH₃)₂,
(r) -CO-CH(NH₂)-CH(CH₃)-CH₂-CH₃,
(s) -CO-CH(NH₂)-CH₂-OH
(t) -CO-CH(NH₂)-CH(OH)-CH₃,
(u) -CO-CH(NH₂)-CH₂-φ,
(v) -CO-CH(NH₂)-CH₂-[*p*-phenyl]-OH,
(w) -CO-CH(NH₂)-CH₂-[2-indolyl]
(x) -CO-CH(NH₂)-CH₂-SH,
(y) -CO-CH(NH₂)-CH₂-CH₂-S-CH₃,
(z) -CO-C∗H-NH-CH₂-CH₂-C∗H₂ where ∗ indicates that the marked carbon atoms are bonded together to form a heterocyclic ring,
(aa) -CO-C∗H-NH-CH₂-CH(OH)-C∗H₂ where ∗ indicates that the marked carbon atoms are bonded together to form a heterocyclic ring,
(bb) -CO-CH(NH₂)-CH₂-COOH,
(cc) -CO-CH(NH₂)-CH₂-CONH₂,
(dd) -CO-CH(NH₂)-CH₂-CH₂-COOH,
(ee) -CO-CH(NH₂)-CH₂-CH₂-CONH₂,
(ff) -CO-CH(NH₂)-CH₂-C∗-NH-CH=N-C∗H= where ∗ indicates that the marked the carbon atoms are bonded together to form a heterocyclic ring,
(gg) -CO-CH(NH₂)-CH₂-CH₂-CH₂-NH-C(= NH)-NH₂,
(hh) -CO-CH(NH₂)-CH₂-CH-₂-CH₂-CH₂-NH₂,
(ii) -CO-CH(NH₂)-CH₂-CH₂-CH(OH)-CH₂-NH₂,
(jj) -CO-CH₂-CH₂-NH₂,
(kk) -CO-CH₂-CH₂-CH₂-NH₂,
(ll) -CO-CH(NH₂)-CH₂-CH₂-CH₂-NH₂,
(mm) -CO-CH(NH₂)-CH₂-CH₂-CH₂-NH-CO-NH₂,
(nn) -CO-CH(NH₂)-CH₂-CH₂-OH, or
(4) -N(R₂₋₁₄)₂ where R₂₋₁₄ may be the same or different and is
(a) C₁-C₆ alkyl optionally substituted with 1 to 3 -OH or -OCH₃,
(b) C₁-C₆ alkylcarbonyl,
(c) C₁-C₆ alkoxycarbonyl,
(d) C₆-C₁₂ arylalkyl,
(e) -φ,
(f) -SO₂-C₁-C₈ alkyl,
(g) CH₃-C*-O-CO-O-C*-CH₂- where ∗ indicates that the marked carbon atoms are attached by a double bond to form a five member ring, and
where R₂₋₂ is
(A) -H, or
(B) C₁-C₈ alkyl optionally substituted with 1 to 4
(1) -F,
(2) -Cl,
(3) -OR₂₋₁₀ where R₂₋₁₀ is as defined above,
(4) -N(R₂₋₁₄)₂ where each R₂₋₁₄ may be the same or different and is as defined above, or
R₂₋₁ and R₂₋₂ are taken together with the attached nitrogen atom to form a heterocyclic ring selected from
(A) 1-pyrrolidinyl optionally substituted on carbon with 1 to 3 R₂₋₃ where R₂₋₃ is selected from
(1) C₁-C₆ alkyl optionally substituted with 1 to 3 -OH or -OCH₃,
(2) C₁-C₆ alkenyl optionally substituted with 1 to 3 -OH or -OCH₃,
(3) C₁-C₆ alkylcarbonyl,
(4) C₁-C₆ alkoxycarbonyl,
(5) C₆-C₁₂ arylalkyl,
(6) =O,
(7) -OH,
(8) -C≡N,
(9) -CO₂R₂₋₄ where R₂₋₄ is
(a) -H,
(b) C₁-C₄ alkyl,
(c) C₆-C₁₂ aryl,
(d) C₆-C₁₂ aralkyl,
(10) -NH₂,
(11) -Cl,
(12) -F,
(13) -Br,
(14) -φ optionally substituted with 1 to 3 -F, -Cl, -Br, -OH, -OCH₃, -OCH₂-φ, -NO₂, C₁-C₃ alkyl, -NH₂, -NHCH₃, N(CH₃)₂, -CO₂R₂₋₄ where R₂₋₄ is as defined above, or
(15) -(CH₂)ₙ₄NR₂₋₆R₂₋₇ where R₂₋₆ and R₂₋₇ are the same or different and are C₁-C₄ alkyl or may taken together with the attached nitrogen atom to form the heterocyclic ring -N*-(CH₂)ₙ₅-R₂₋₈-(CH₂)ₙ₆* where ∗ indicates that the marked atoms are bonded together resulting in the formation of a ring, where n₄ is 0 to 3, n₅ is 1 to 5, n₆ is 0 to 3 and R₂₋₈ is
(a) -CH₂-,
(b) -O-,
(c) -S-, or
(d) -NR₂₋₄ where R₂₋₄ is as defined above,
(B) 1-piperdinyl optionally substituted on carbon with 1 to 3 R₂₋₃ where R₂₋₃ is as defined above,
(C) 1-morpholinyl optionally substituted on carbon with 1 to 3 R₂₋₃ where R₂₋₃ is as defined above,
(D) 1-piperazinyl optionally substituted on carbon with 1 to 3 R₂₋₃ where R₂₋₃ is as defined above and optionally substituted in the 4-position with R₂₋₅ where R₂₋₅ is
(1) C₁-C₆ alkyl optionally substituted with 1 to 3 -OH or -OCH₃,
(2) C₁-C₆ alkylcarbonyl,
(3) C₁-C₆ alkoxycarbonyl,
(4) C₆-C₁₂ arylalkyl,
(5) -φ,
(6) -SO₂-C₁-C₈ alkyl,
(7) CH₃-C*-O-CO-O-C*-CH₂- where ∗ indicates that the marked carbon atoms are attached by a double bond to form a five member ring,
(E) 1-aziridinyl optionally substituted on carbon with 1 to 2 R₂₋₃ where R₂₋₃ is as defined above,
(F) 1-azetidinyl optionally substituted on carbon with 1 to 3 R₂₋₃ where R₂₋₃ is as defined above,
(G) 1-hexamethyleneimino optionally substituted on carbon with 1 to 3 R₂₋₃ where R₂₋₃ is as defined above,
(H) 1-pyrrolyl optionally substituted on carbon with 1 to 3 R₂₋₃ where R₂₋₃ is as defined above,
(I) 1-imidazolyl optionally substituted on carbon with 1 to 3 R₂₋₃ where R₂₋₃ is as defined above,
(J) 1-pyrazoyl optionally substituted on carbon with 1 to 3 R₂₋₃ where R₂₋₃ is as defined above,
(K) 1-pyrazolidinyl optionally substituted on carbon with 1 to 3 R₂₋₃ where R₂₋₃ is as defined above,
(L) 1,2,3-triazolyl optionally substituted on carbon with 1 to 3 R₂₋₃ where R₂₋₃ is as defined above,
(M) 1,2,4-triazolyl optionally substituted on carbon with 1 to 3 R₂₋₃ where R₂₋₃ is as defined above,
(N) 1-tetrazolyl optionally substituted on carbon with 1 to 3 R₂₋₃ where R₂₋₃ is as defined above,
(O) 1-thiomorpholinyl optionally substituted on carbon with 1 to 3 R₂₋₃ where R₂₋₃ is as defined above,
(P) 1-thiazolidinyl optionally substituted on carbon with 1 to 3 R₂₋₃ where R₂₋₃ is as defined above, 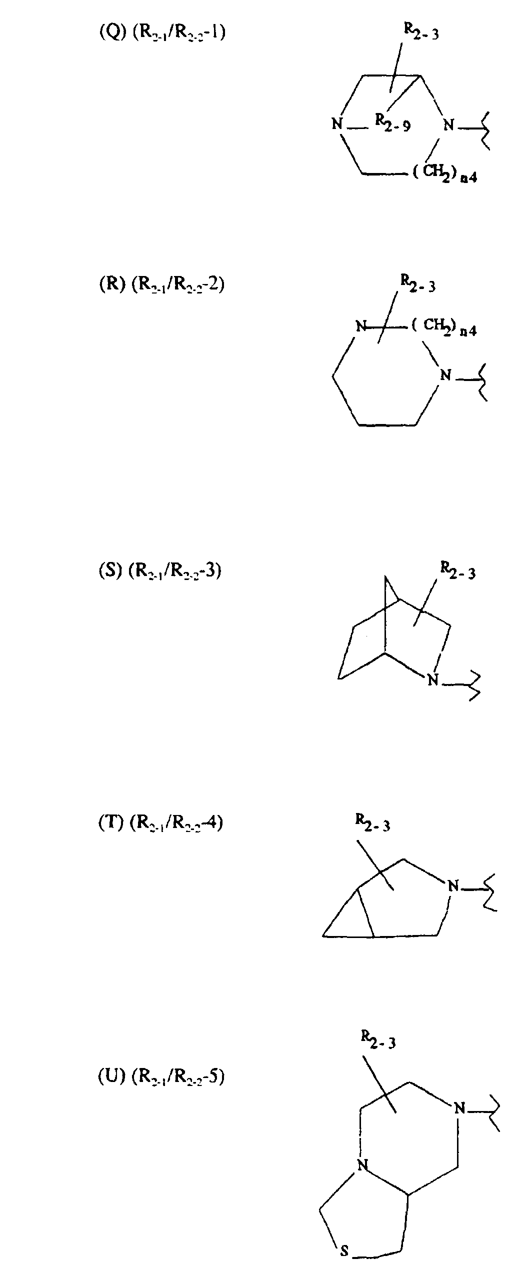 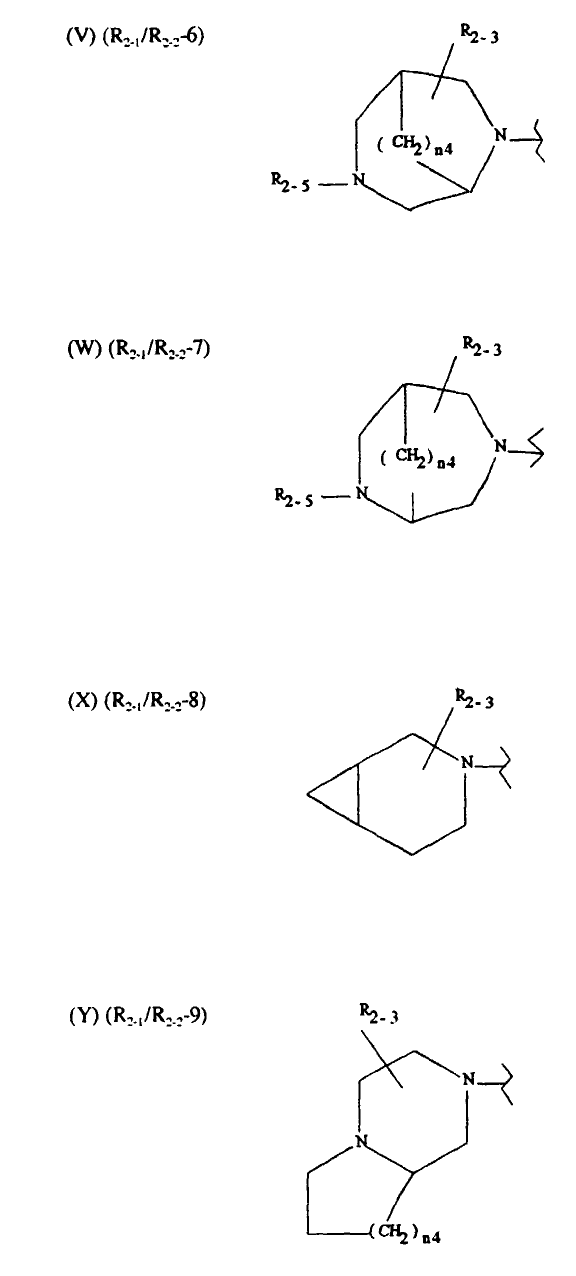 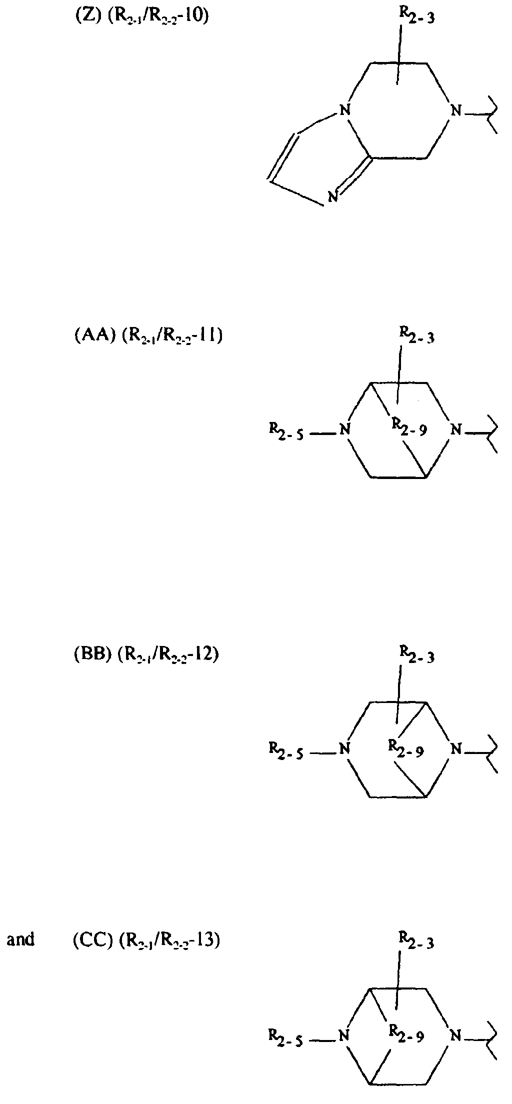
where R₂₋₃ and R₂₋₅ are as defined above, and
where R₂₋₉ is
(A) -(CH₂)ₙ₄ where n₄ is 1 to 3,
(B) -CH₂OCH₂,
(C) -CH₂SCH₂,
(D) -CH₂SO₂CH₂,
(E) -CH₂S,
(F) -CH₂SO₂, or
(G) -CH₂N(R₂₋₅)CH₂ where R₂₋₅ is as defined above;
with the proviso that R₂₋₁ and R₂₋₂ cannot both be -H;
where R₄₋₁ is as defined for R₂₋₁, and may be the same as or different from R₂₋₁,
where R₄₋₂ is as defined bar R₂₋₂, and may be the same as or different from R₂₋₂, with the proviso that R₄₋₁ and R₄₋₂ can not both be -H; and
where R₇ is
(A) -H,
(B) C₁-C₈ alkyl optionally substituted with 1 to 4 R₇₋₁ where R₇₋₁ is
(1) -F, -Cl, -Br,
(2) C₁-C₄ alkyl,
(3) -CF₃,
(4) -φ,
(5) -OR₇₋₂ where R₇₋₂ is
(a) -H,
(b) C₁-C₄ alkyl,
(c) phosphate,
(d) sulfate,
(e) -CO-R₇₋₈ where R₇₋₈ is C₁-C₄ alkyl or C₆-C₉ aralkyl,
(f) -CO-NR₇₋₁₀R₇₋₁₁ where R₇₋₁₀ and R₇₋₁₁ are the same or different and are -H or C₁-C₃ alkyl,
(g) sulfamate,
(h) glucosyl,
(i) galactosyl,
(j) glucuronic acid,
(k) maltosyl,
(l) arabinosyl,
(m) xylosyl,
(n) -CO-CH(NH₂)-H,
(o) -CO-CH(NH₂)-CH₃,
(p) -CO-CH(NH₂)-CH(CH₃)₂,
(q) -CO-CH(NH₂)-CH₂-CH(CH₃)₂,
(r) -CO-CH(NH₂)-CH(CH₃)-CH₂-CH₃,
(s) -CO-CH(NH₂)-CH₂-OH
(t) -CO-CH(NH₂)-CH(OH)-CH₃,
(u) -CO-CH(NH₂)-CH₂-φ,
(v) -CO-CH(NH₂)-CH₂-[*p*-phenyl]-OH,
(w) -CO-CH(NH₂)-CH₂-[2-indolyl]
(x) -CO-CH(NH₂)-CH₂-SH,
(y) -CO-CH(NH₂)CH₂-CH₂-S-CH₃,
(z) -CO-C*H-NH-CH₂-CH₂-C*H₂ where ∗ indicates that the marked carbon atoms are bonded together to form a heterocyclic ring,
(aa) -CO-C*H-NH-CH₂-CH(OH)-C*H₂ where ∗ indicates that the marked carbon atoms are bonded together to form a heterocyclic ring,
(bb) -CO-CH(NH₂)-CH₂-COOH,
(cc) -CO-CH(NH₂)-CH₂-CONH₂,
(dd) -CO-CH(NH₂)-CH₂-CH₂-COOH,
(ee) -CO-CH(NH₂)-CH₂-CH₂-CONH₂,
(ff) -CO-CH(NH₂)-CH₂-C*-NH-CH=N-C*H= where ∗ indicates that the marked carbon atoms are bonded together to form a heterocyclic ring,
(gg) -CO-CH(NH₂)-CH₂-CH₂-CH₂-NH-C(=NH)-NH₂,
(hh) -CO-CH(NH₂)-CH₂-CH₂-CH₂-CH₂-NH₂,
(ii) -CO-CH(NH₂)-CH₂-CH₂-CH(OH)-CH₂-NH₂,
(jj) -CO-CH₂-CH₂-NH₂,
(kk) -CO-CH₂-CH₂-CH₂-NH₂,
(ll) -CO-CH(NH₂)-CH₂-CH₂-CH₂-NH₂,
(mm) -CO-CH(NH₂)-CH₂-CH₂-CH₂-NH-CO-NH₂,
(nn) -CO-CH(NH₂)-CH₂-CH₂-OH,
(6) -SR₇₋₂ where R₇₋₂ is defined above,
(7) -NHR₇₋₃ where R₇₋₃ is -H or C₁-C₄ alkyl,
(8) -NR₇₋₄R₇₋₅ where R₇₋₄ and R₇₋₅ are the same or different and are C₁-C₄
alkyl or may taken together with the attached nitrogen atom to form
-N*-(CH₂)ₙ₁-R₅₋₆-(CH₂)ₙ₂* where ∗ indicates that the marked atoms are bonded together resulting in the formation of a ring, where n₁ is 1 to 5, n₂ is 0 to 3 and R₅₋₆ is
(a) -CH₂-,
(b) -O-,
(c) -S-,
(d) -NR₇₋₉ where R₇₋₉ is
(i) C₁-C₆ alkyl optionally substituted with 1 to 3 -OH or -OCH₃,
(ii) C₁-C₆ alkylcarbonyl,
(iii) C₁-C₆ alkoxycarbonyl,
(iv) C₆-C₁₂ arylalkyl,
(v) -φ,
(vi) -SO₂-C₁-C₈ alkyl,
(vii) CH₃-C∗-O-CO-O-C∗-CH₂- where ∗ indicates that the marked carbon atoms are attached by a double bond to form a five member ring,
(9) -(CH₂)ₙ₃CO₂R₇₋₂, where n₃ is 0 to 6 and R₇₋₂ is as defined above,
(10) -(CH₂)ₙ₃CON(R₇₋₃)₂ where n₃ is as defined as above and where each R₇₋₃ may be the same or different and is defined above,
(11) -(CH₂)ₙ₃CONR₇₋₄R₇₋₅ where n₃, R₇₋₄, R₇₋₅ are as defined above,
(12) -(CH₂)ₙ₁OR₇₋₂ where R₇₋₂ and n₁ are as defined above,
(13) -(CH₂)ₙ₁OCOR₇₋₃ where R₇₋₃ and n₁ are as defined above,
(14) -(CH₂)ₙ₁SR₇₋₂ where R₇₋₂ and n₁ are as defined above,
(15) -(CH₂)ₙ₁NHR₇₋₃ where R₇₋₃ and n₁ are as defined above,
(16) -(CH₂)ₙ₁NR₇₋₄R₇₋₅ where R₇₋₄, R₇₋₅, and n₁ are as defined above,
(C) -(CH₂)ₙ₃-φ optionally substituted with 1 to 4 R₇₋₁ where R₇₋₁ and n₃ are as defined as above.
(D) -(CH₂)ₙ₃-pyridin-2-, 3- or 4-yl optionally substituted with 1 to 4 R₇₋₁ where n₃ and R₇₋₁ are as defined above,
(E) -(CH₂)ₙ₃-naphthalin-1- or 2-yl optionally substituted with 1 to 4 R₇₋₁ where n₃ and R₇₋₁ are as defined above,
(F) -(CH₂)ₙ₃CO₂R₇₋₂ where n₃ and R₇₋₃ are as defined above,
(G) -(CH₂)ₙ₃CON(R₇₋₃)₂ where n₃ is as defined as above and where each R₇₋₃ may be the same or different and is as defined above,
(H) -(CH₂)ₙ₃CONR₇₋₄R₇₋₅ where n₃, R₇₋₄ and R₇₋₅ are as defined above,
(I) -(CH₂)ₙ₃SO₃R₇₋₂ where n₃ and R₇₋₂ are as defined above, or
(J) C₃-C₇ cycloalkyl; or a pharmaceutically-acceptable salt thereof.

2. A tricyclic amine of formula XXX as defined in claim 1, with the exception of 6,7-dimethoxy-2,4-di(1-piperidinyl)-9H-pyrimido[4,5-b]indole and 6,7-dimethoxy-2,4-di(4-methyl-1-piperazinyl)-9H-pyrimido[4,5-b]indole.

3. A tricyclic amine according to claim 2, where R₂₋₁ and R₂₋₂ are taken together with the attached nitrogen atom to form 1-pyrrolidinyl, 1-piperazinyl, 1-thiomorpholinyl or 4-methylpiperazin-1-yl.

4. A tricyclic amine according to claim 3, where R₂₋₁ and R₂₋₂ together are 1-pyrrolidinyl and 1-piperazinyl.

5. A tricyclic amine according to claim 2, where R₄₋₁ and R₄₋₂ are taken together with the attached nitrogen atom to form 1-pyrrolidinyl, 1-piperazinyl, 1-thiomorpholinyl or 4-methylpiperazin-1-yl.

6. A tricyclic amine according to claim 5, where R₄₋₁ and R₄₋₂ together are 1-pyrrolidinyl and 1-piperazinyl.

7. A tricyclic amine according to claim 2, where R₇ is -H, -CH₃, phenyl, 2-(1-morpholinyl)ethyl or 2-(1-piperazinyl)ethyl.

8. A tricyclic amine according to claim 2, in the form of a salt with an acid selected from hydrochloric, hydrobromic, methanesulfonic, sulfuric, phosphoric, nitric, benzoic, citric, tartaric, fumaric and maleic acids, CH₃-(CH₂)₀₋₄-COOH and HOOC-(CH₂)₀₋₄COOH.

9. A tricyclic amine according to claim 2, selected from
5,6,7,8-tetrahydro-9-methyl-2,4-di-(1-pyrrolidinyl)-5H-pyrimido[4,5-b]indole,
9-methyl-2,4-di-(1-pyrrolidinyl)-9H-pyrimadol[4,5-b]indole,
5,6,7,8-tetrahydro-2,4-di-(1-pyrrolidinyl)-1H-pyrimido[4,5-b]indole, and
5,6,7,8-tetrahydro-9-[2-(1-piperazinyl)ethyl]-2,4-di-(1-pyrrolidinyl)-5H-pyrimido[4,5-b]indole.

## Patentansprüche

1. Verwendung eines tricyclischen heterocyclischen Amins der Formel: 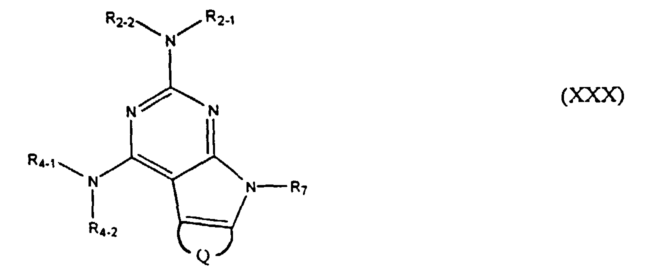 worin bedeuten:
**Q** -(CH₂)₃₋₅ oder -CR₅₆₋₁=CR₅₆₋₂-CR₅₆₋₃=CR₅₆₋₄ mit R₅₆₋₁, R₆₋₂, R₅₆₋₃ und R₅₆₋₄ gleich -H, -F, -Cl, -Br, -OH, -OCH₃, -OCH₂-φ, -NO₂, C₁-C₃-Alkyl, -NH₂, -NHCH₃, N(CH₃)₂ oder -CO₂R₅₆₋₅, worin R₅₆₋₅ ist -H, C₁-C₄-Alkyl, C₆-C₁₂-Aryl oder C₆-C₁₂-Aralkyl,
**R**_{**2-1**}
(A) -H oder
(B) C₁-C₈-Alkyl, ggf. substituiert durch 1 bis 4
(1) -F,
(2) -Cl,
(3) -OR₂₋₁₀, worin R₂₋₁₀ ist
(a) -H,
(b) C₁-C₄-Alkyl,
(c) Phosphat,
(d) Sulfat,
(e) -CO-R₂₋₁₁ mit R₂₋₁₁ gleich C₁-C₄-Alkyl oder C₆-C₉-Aralkyl,
(f) -CO-NR₂₋₁₂NR₂₋₁₃ mit R₂₋₁₂ und R₂₋₁₃, die gleich oder verschieden sind, gleich -H oder C₁-C₃-Alkyl,
(g) Sulfamat,
(h) Glucosyl,
(i) Galactosyl,
(j) Glucoronsäure,
(k) Maltosyl,
(l) Arabinosyl,
(m) Xylosyl,
(n) -CO-CH(NH₂)-H,
(o) -CO-CH(NH₂)-CH₃,
(p) -CO-CH(NH₂)-CH(CH₃)_{2,}
(q) -CO-CH(NH₂)-CH₂-CH(CH₃)₂,
(r) -CO-CH(NH₂)-CH(CH₃)-CH₂-CH₃,
(s) -CO-CH(NH₂)-CH₂-OH,
(t) -CO-CH(NH₂)-CH(OH)-CH_{3,}
(u) -CO-CH(NH₂)-CH₂-φ,
(v) -CO-CH(NH₂)-CH₂-[p-Phenyl]-OH,
(w) -CO-CH(NH₂)-CH₂-[2-Indolyl],
(x) -CO-CH(NH₂)-CH₂-SH,
(y) -CO-CH(NH₂)-CH₂-CH₂-S-CH₃,
(z) -CO-C*H-NH-CH₂-CH₂-C*H₂, worin * anzeigt, daß die (damit) gekennzeichneten Kohlenstoffatome miteinander unter Bildung eines heterocyclischen Rings verbunden sind,
(aa) -CO-C*H-NH-CH₂-CH(OH)-C*H₂, worin * anzeigt, daß die (damit) gekennzeichneten Kohlenstoffatome miteinander unter Bildung eines heterocyclischen Rings verbunden sind,
(bb) -CO-CH(NH₂)-CH₂-COOH,
(cc) -CO-CH(NH₂)-CH₂-CONH₂,
(dd) -CO-CH(NH₂)-CH₂-CH₂-COOH,
(ee) -CO-CH(NH₂)-CH₂-CH₂-CONH₂,
(ff) -CO-CH(NH₂)-CH₂-C*-NH-CH=N-C*H=, worin * anzeigt, daß die (damit) gekennzeichneten Kohlenstoffatome miteinander unter Bildung eines heterocyclischen Rings verbunden sind,
(gg) -CO-CH(NH₂)-CH₂-CH₂-CH₂-NH-C(=NH)-NH₂,
(hh) -CO-CH(NH₂)-CH₂-CH₂-CH₂-CH₂-NH₂,
(ii) -CO-CH(NH₂)-CH₂-CH₂-CH(OH)-CH₂-NH₂,
(jj) -CO-CH₂-CH₂-NH₂,
(kk) -CO-CH₂-CH₂-CH₂-NH₂,
(ll) -CO-CH(NH₂)-CH₂-CH₂-CH₂-NH₂,
(mm) -CO-CH(NH₂)-CH₂-CH₂-CH₂-NH-CO-NH₂
(nn) -CO-CH(NH₂)-CH₂-CH₂-OH oder
(4) -N(R₂₋₁₄)₂, worin R₂₋₁₄ gleich oder verschieden sein kann und bedeutet:
(a) C₁-C₆-Alkyl, ggf. substuiert durch 1 bis 3 -OH oder -OCH₃
(b) C₁-C₆-Alkylcarbonyl,
(c) C₁-C₆-Alkoxycarbonyl,
(d) C₆-C₁₂-Arylalkyl,
(e) -φ,
(f) -SO₂-C₁-C₈-Alkyl
(g) CH₃-C*-O-CO-O-C*-CH₂-, worin * anzeigt, daß die (damit) gekennzeichneten Kohlenstoffatome über eine Doppelbindung unter Bildung eines 5-gliedrigen Rings aneinander gebunden sind, und
**R**_{**2-2**}
(A) -H oder
(B) C₁-C₈-Alkyl, ggf. substituiert durch 1 bis 4
(1) -F,
(2) -Cl,
(3) -OR₂₋₁₀ mit R₂₋₁₀ in der zuvor angegebenen Definition,
(4) -N(R₂₋₁₄)₂, worin die einzelnen Reste R₂₋₁₄ gleich oder verschieden sein können und obiger Definition entsprechen, oder
**R**_{**2-1**} und **R**_{**2-2**} zusammen mit dem Stickstoffatom, an dem sie hängen, einen heterocyclischen Ring, ausgewählt aus
(A) 1-Pyrrolidinyl, ggf. substituiert am Kohlenstoff durch 1 bis 3 R₂₋₃, wobei R₂₋₃ ausgewählt ist aus:
(1) C₁-C₆-Alkyl, ggf. substituiert mit 1 bis 3 -OH oder -OCH₃,
(2) C₁-C₆-Alkenyl, ggf. substituiert mit 1 bis 3 -OH oder -OCH₃,
(3) C₁-C₆-Alkylcarbonyl
(4) C₁-C₆-Alkoxycarbonyl
(5) C₆-C₁₂-Aralkyl,
(6) =O,
(7) -OH,
(8) -C≡N,
(9) -CO₂R₂₋₄ mit R₂₋₄ gleich
(a) -H,
(b) C₁-C₄-Alkyl,
(c) C₆-C₁₂-Aryl,
(d) C₆-C₁₂-Aralkyl,
(10) -NH₂,
(11) -Cl,
(12) -F,
(13) -Br,
(14) -φ ggf. substituiert durch 1 bis 3 -F, -Cl, -Br, -OH, -OCH₃, -OCH₂-φ, -NO₂, C₁-C₃-Alkyl, -NH₂, -NHCH₃, N(CH₃)₂, -CO₂R₂₋₄ mit R₂₋₄ in der zuvor angegebenen Definition, oder
(15) -(CH₂)ₙ₄NR₂₋₆R₂₋₇, wobei R₂₋₆ und R₂₋₇ gleich oder verschieden sind und C₁-C₄-Alkyl bedeuten oder zusammen mit dem Stickstoffatom, an dem sie hängen, einen heterocyclischen Ring -N*-(CH₂)ₙ₅-R₂₋₈-(CH₂)ₙ₆*, wobei * anzeigt, daß die (damit) markierten Atome miteinander unter Ringbildung verbunden sind, wobei n₄ = 0 bis 3, n₅ = 1 bis 5, n₆ = 0 bis 3 und R₂₋₈ ist
(a) -CH₂-,
(b) -O-,
(c) -S- oder
(d) -NR₂₋₄ mit R₂₋₄ in der zuvor angegebenen Definition;
(B) 1-Piperidinyl, ggf. substituiert am Kohlenstoff durch 1 bis 3 R₂₋₃ mit R₂₋₃ in der oben angegebenen Definition;
(C) 1-Morpholinyl, ggf. substituiert am Kohlenstoff durch 1 bis 3 R₂₋₃ mit R₂₋₃ in der zuvor angegebenen Definition;
(D) 1-Piperazinyl, ggf. substituiert am Kohlenstoff durch 1 bis 3 R₂₋₃ mit R₂₋₃ in der zuvor angegebenen Definition und ggf. substituiert in 4-Stellung durch R₂₋₅ mit R₂₋₅ gleich
(1) C₁-C₆-Alkyl, ggf. substituiert durch 1 bis 3 -OH oder -OCH₃,
(2) C₁-C₆-Alkylcarbonyl,
(3) C₁-C₆-Alkoxycarbonyl,
(4) C₆-C₁₂-Arylalkyl,
(5) -φ,
(6) -SO₂-C₁-C₈-Alkyl,
(7) CH₃-C*-O-CO-O-C*-CH₂-, worin * anzeigt, daß die (damit) markierten Kohlenstoffatome miteinander über eine Doppelbindung unter Bildung eines 5-gliedrigen Rings verbunden sind;
(E) 1-Aziridinyl, ggf. substituiert am Kohlenstoff durch 1 bis 2 R₂₋₃ mit R₂₋₃ in der zuvor angegebenen Definition;
(F) 1-Azetidinyl, ggf. substituiert am Kohlenstoff durch 1 bis 3 R₂₋₃ mit R₂₋₃ in der zuvor angegebenen Definition;
(G) 1-Hexamethylenimino, ggf. substituiert am Kohlenstoff durch 1 bis 3 R₂₋₃ mit R₂₋₃ in der zuvor angegebenen Definition;
(H) 1-Pyrrolyl, ggf. substituiert am Kohlenstoff durch 1 bis 3 R₂₋₃ mit R₂₋₃ in der zuvor angegebenen Definition;
(I) 1-Imidazolyl, ggf. substituiert am Kohlenstoff durch 1 bis 3 R₂₋₃ mit R₂₋₃ in der zuvor angegebenen Definition;
(J) 1-Pyrazoyl, ggf. substituiert am Kohlenstoff durch 1 bis 3 R₂₋₃ mit R₂₋₃ in der zuvor angegebenen Definition;
(K) 1-Pyrazolidinyl, ggf. substituiert am Kohlenstoff durch 1 bis 3 R₂₋₃ mit R₂₋₃ in der zuvor angegebenen Definition;
(L) 1,2,3-Triazolyl, ggf. substituiert am Kohlenstoff durch 1 bis 3 R₂₋₃ mit R₂₋₃ in der zuvor angegebenen Definition;
(M) 1,2,4-Triazolyl, ggf. substituiert am Kohlenstoff durch 1 bis 3 R₂₋₃ mit R₂₋₃ in der zuvor angegebenen Definition;
(N) 1-Tetrazolyl, ggf. substituiert am Kohlenstoff durch 1 bis 3 R₂₋₃ mit R₂₋₃ in der zuvor angegebenen Definition;
(O) 1-Thiomorpholinyl, ggf. substituiert am Kohlenstoff durch 1 bis 3 R₂₋₃ mit R₂₋₃ in der zuvor angegebenen Definition;
(P) 1-Thiazolidinyl, ggf. substituiert am Kohlenstoff durch 1 bis 3 R₂₋₃ mit R₂₋₃ in der zuvor angegebenen Definition; 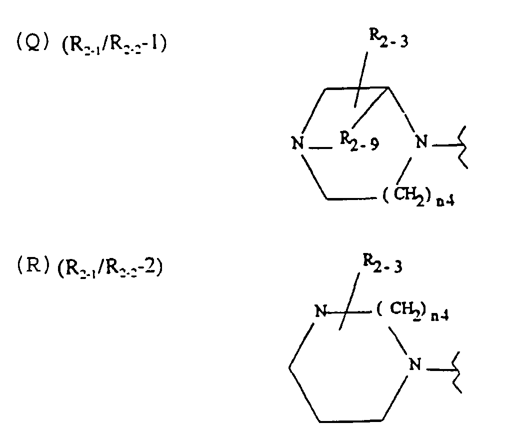 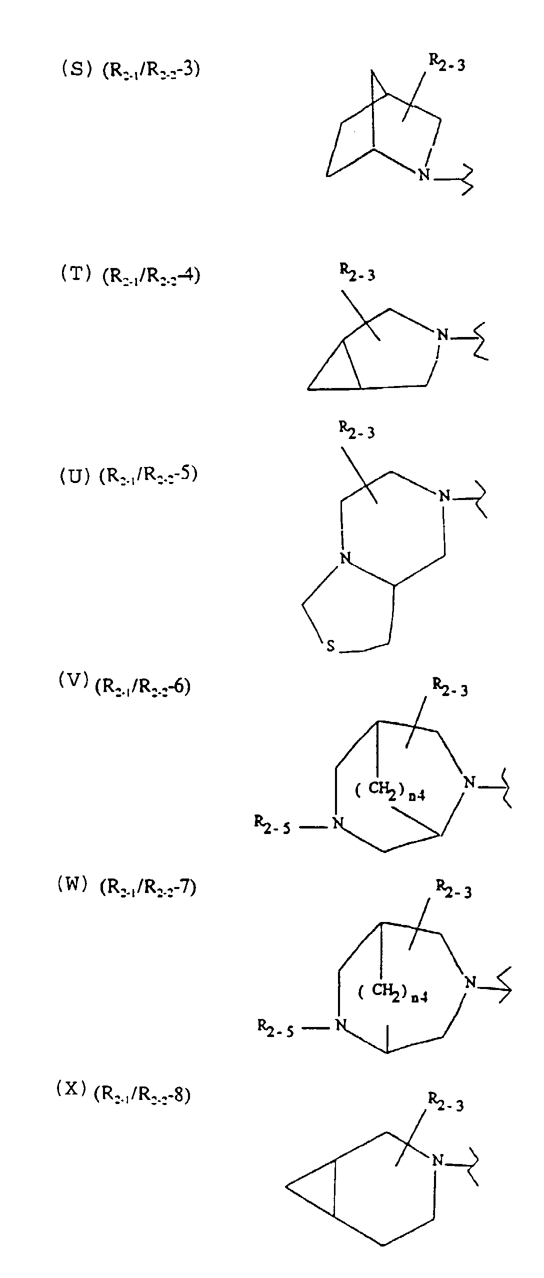 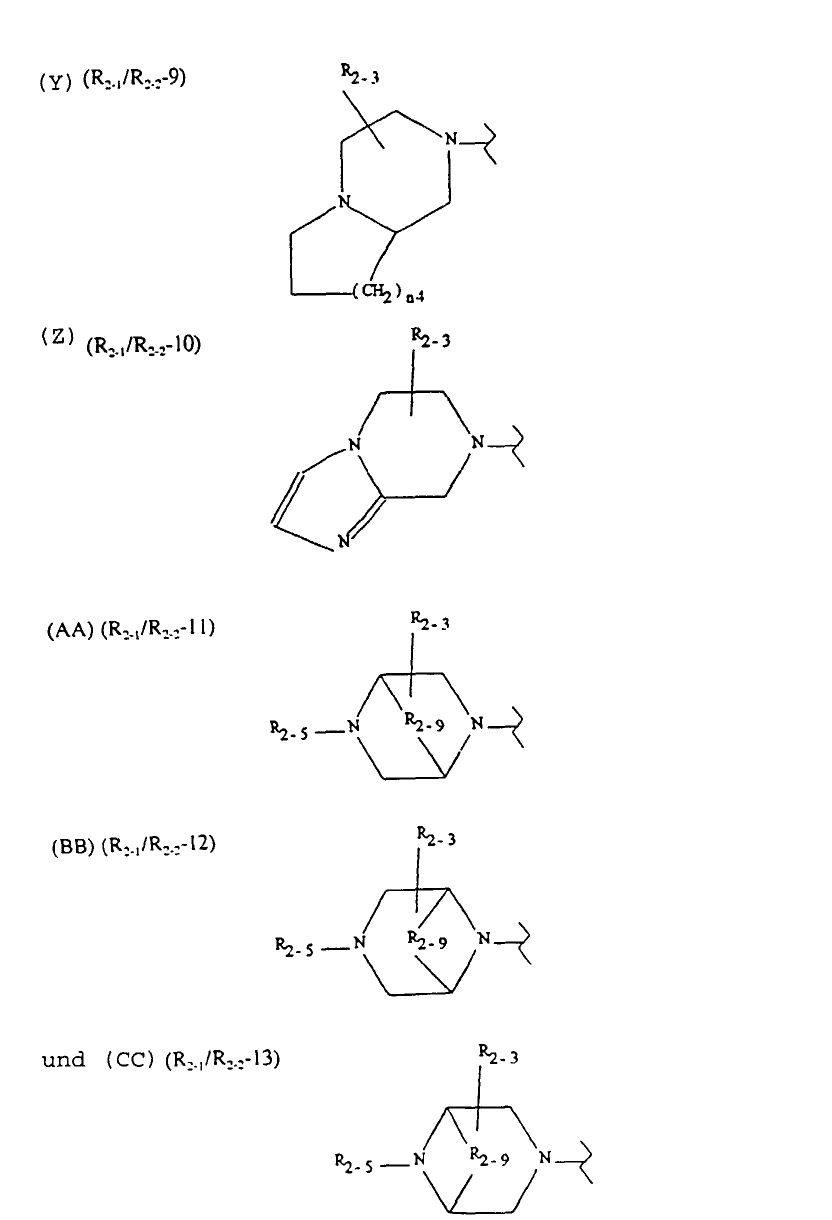 worin R₂₋₃ und R₂₋₅ den zuvor angegebenen Definitionen entsprechen, und
worin R₂₋₉ ist
(A) -(CH₂)ₙ₄ mit n₄ = 1 bis 3
(B) -CH₂OCH₂,
(C) -CH₂SCH₂,
(D) -CH₂SO₂CH₂,
(E) -CH₂S,
(F) -CH₂SO₂ oder
(G) -CH₂N(R₂₋₅)CH₂ mit R₂₋₅ in der zuvor angegebenen Definition;
wobei gilt, daß nicht beide Reste R₂₋₁ und R₂₋₂ gleichzeitig -H darstellen;
wobei R₄₋₁ der Definition von R₂₋₁ entspricht und denselben Rest wie R₂₋₁ darstellen oder von diesem verschieden sein kann,
worin R₄₋₂ der Definition von R₂₋₂ entspricht und denselben Rest wie R₂₋₂ bedeuten oder von diesem verschieden sein kann, wobei gilt, daß R₄₋₁ und R₄₋₂ nicht gleichzeitig für -H stehen können; und
**R**_{**7**}
(A) -H,
(B) C₁-C₈-Alkyl, ggf. substituiert durch 1 bis 4 R₇₋₁ mit R₇₋₁ gleich
(1) -F, -Cl, -Br,
(2) C₁-C₄-Alkyl,
(3) -CF₃,
(4) -φ,
(5) -OR₇₋₂, worin R₇₋₂ ist
(a) -H,
(b) C₁-C₄-Alkyl,
(c) Phosphat,
(d) Sulfat,
(e) -CO-R₇₋₈, worin R₇₋₈ ist C₁-C₄-Alkyl oder C₆-C₉-Aralkyl,
(f) -CO-NR₇₋₁₀R₇₋₁₁, worin R₇₋₁₀ und R₇₋₁₁, die gleich oder verschieden sind, -H oder C₁-C₃-Alkyl bedeuten,
(g) Sulfamat,
(h) Glucosyl,
(i) Galactosyl,
(j) Glucoronsäure,
(k) Maltosyl,
(l) Arabinosyl,
(m) Xylosyl,
(n) -CO-CH(NH₂)-H,
(o) -CO-CH(NH₂)-CH₃,
(p) -CO-CH(NH₂)-CH(CH₃)_{2,}
(q) -CO-CH(NH₂)-CH₂-CH(CH₃)₂,
(r) -CO-CH(NH₂)-CH(CH₃)-CH₂-CH₃,
(s) -CO-CH(NH₂)-CH₂-OH,
(t) -CO-CH(NH₂)-CH(OH)-CH_{3,}
(u) -CO-CH(NH₂)-CH₂-φ,
(v) -CO-CH(NH₂)-CH₂-[p-Phenyl]-OH,
(w) -CO-CH(NH₂)-CH₂-[2-Indolyl],
(x) -CO-CH(NH₂)-CH₂-SH,
(y) -CO-CH(NH₂)-CH₂-CH₂-S-CH₃,
(z) -CO-C*H-NH-CH₂-CH₂-C*H₂, worin * anzeigt, daß die (damit) gekennzeichneten Kohlenstofffatome miteinander unter Bildung eines heterocyclischen Rings verbunden sind,
(aa) -CO-C*H-NH-CH₂-CH(OH)-C*H₂, worin * anzeigt, daß die (damit) gekennzeichneten Kohlenstoffatome miteinander unter Bildung eines heterocyclischen Rings verbunden sind,
(bb) -CO-CH(NH₂)-CH₂-COOH,
(cc) -CO-CH(NH₂)-CH₂-CONH₂,
(dd) -CO-CH(NH₂)-CH₂-CH₂-COOH,
(ee) -CO-CH(NH₂)-CH₂-CH₂-CONH₂,
(ff) -CO-CH(NH₂)-CH₂-C*-NH-CH=N-C*H=, worin * anzeigt, daß die (damit) gekennzeichneten Kohlenstoffatome miteinander unter Bildung eines heterocyclischen Rings verbunden sind,
(gg) -CO-CH(NH₂)-CH₂-CH₂-CH₂-NH-C(=NH)-NH₂,
(hh) -CO-CH(NH₂)-CH₂-CH₂-CH₂-CH₂-NH₂,
(ii) -CO-CH(NH₂)-CH₂-CH₂-CH(OH)-CH₂-NH₂,
(jj) -CO-CH₂-CH₂-NH₂,
(kk) -CO-CH₂-CH₂-CH₂-NH₂,
(ll) -CO-CH(NH₂)-CH₂-CH₂-CH₂-NH₂,
(mm) -CO-CH(NH₂)-CH₂-CH₂-CH₂-NH-CO-NH₂
(nn) -CO-CH(NH₂)-CH₂-CH₂-OH oder
(6) -SR₇₋₂ mit R₇₋₂ in der zuvor angegebenen Definition,
(7) -NHR₇₋₃ mit R₇₋₃ gleich -H oder C₁-C₄-Alkyl,
(8) -NR₇₋₄R₇-₅, worin R₇₋₄ und R₇₋₅, die gleich oder verschieden sind, für C₁-C₄-Alkyl stehen oder zusammen mit dem Stickstoffatom, an dem sie hängen, -N*-(CH₂)ₙ₁-R₅₋₆-(CH)ₙ₂, wobei * anzeigt, daß die (damit) markierten Atome miteinander unter Ringbildung verbunden sind, wobei n₁ = 1 bis 5, n₂ = 0 bis 3 und R₅₋₆ ist
(a) -CH₂-,
(b) -O-,
(c) -S-,
(d) -NR₇₋₉ mit R₇₋₉ gleich
(i) C₁-C₆-Alkyl, ggf. substituiert mit 1 bis 3 -OH oder -OCH₃,
(ii) C₁-C₆-Alkylcarbonyl,
(iii) C₁-C₆-Alkoxycarbonyl
(iv) C₆-C₁₂-Arylalkyl,
(v) -φ,
(vi) -SO₂-C₁-C₈-Alkyl,
(vii) CH₃-C*-O-CO-O-C*-CH₂-, worin * anzeigt, daß die (damit) markierten Kohlenstoffatome über eine Doppelbindung unter Bildung eines 5-gliedrigen Rings miteinander verbunden sind,
(9) -(CH₂)ₙ₃CO₂R₂₋₇ mit n₃ = 0 bis 6 und R₇₋₂ in der zuvor angegebenen Definition,
(10) -(CH₂)ₙ₃CON(R₇₋₃)₂ mit n₃ in der zuvor angegebenen Definition, wobei die einzelnen Reste R₇₋₃ gleich oder verschieden sein können und unter die obige Definition fallen,
(11) -(CH₂)ₙ₃CONR₇₋₄R₇₋₅ mit n₃, R₇₋₄ und R₇₋₅ in der zuvor angegebenen Definition,
(12) -(CH₂)ₙ₁OR₇₋₂ mit R₇₋₂ und n₁ in der zuvor angegebenen Definition,
(13) -(CH₂)ₙ₁OCOR₇₋₃ mit R₇₋₃ und n₁ in der zuvor angegebenen Definition,
(14) -(CH₂)n₁SR₇₋₂ mit R₇₋₂ und n₁ in der zuvor angegebenen Definition,
(15) -(CH₂)ₙ₁NHR₇₋₃ mit R₇₋₃ und n₁ in der zuvor angegebenen Definition,
(16) -(CH₂)ₙ₁NR₇₋₄R₇₋₅ mit R₇₋₄, R₇₋₅ und n₁ in der zuvor angegebenen Definition,
(C) -(CH₂)ₙ₃-φ, ggf. substituiert durch 1 bis 4 R₇₋₁ mit R₇₋₁ und n₃ in der zuvor angegebenen Definition,
(D) -(CH₂)ₙ₃-Pyridin-2-, -3- oder -4-yl, ggf. substituiert durch 1 bis 4 R₇₋₁ mit n₃ und R₇₋₁ in der zuvor angegebenen Definition,
(E) -(CH₂)ₙ₃-Naphthalin-1- oder -2-yl, ggf. substituiert durch 1 bis 4 R₇₋₁ mit n₃ und R₇₋₁ in der zuvor angegebenen Definition,
(F) -(CH₂)ₙ₃CO₂R₇₋₂ mit n₃ und R₇₋₂ in der zuvor angegebenen Definition,
(G) -(CH₂)ₙ₃CON(R₇₋₃)₂ mit n₃ in der zuvor angegebenen Definition, wobei die einzelnen Reste R₇₋₃ gleich oder verschieden sein können und unter die zuvor angegebene Definition fallen,
(H) -(CH₂)ₙ₃CONR₇₋₄R₇₋₅ mit n₃, R₇₋₄ und R₇₋₅ in der zuvor angegebenen Definition,
(I) -(CH₂)ₙ₃SO₃R₇₋₂ mit n₃ und R₇₋₂ in der zuvor angegebenen Definition, oder
(J) C₃-C₇-Cycloalkyl,
oder eines pharmazeutisch akzeptablen Salzes hiervon zur Herstellung eines Medikaments zur Verwendung bei der Behandlung/Verhinderung von Wirbelsäulenverletzungen, milden und/oder mäßigen bis schweren Kopfverletzungen, subarachnoidaler Blutung und anschließendem ischämischem (thromboembolischem) Schlaganfall, Asthma und verminderter Schleimbildung/-sekretion in der Lunge, Muskeldystrophie, Adriamycin-Herztoxizität, Parkinsonismus, Alzheimer'scher Krankheit, sonstiger degenerativer neurologischer Störungen, multipler Sklerose, Organschädigung während der Reperfusion nach einer Transplantation, Hauttransplantatabstoßung, hämorrhagischem, traumatischem und septischem Schock und Affektionen, z.B. schweren Verbrennungen, ARDS, entzündlichen Erkrankungen, wie Knochenentzündung oder primär chronischer Polyarthritis, nephrotischem Syndrom (immunologisch), systemischem Lupus Erythematosis, allergischen Reaktionen, Diabetes, Atherosklerose, Entzündungen (dermatologische, entzündungshemmende und Antipsoriasis-Mittel), Emphysem, Krebs (begrenzt eine Metastasierung, begrenzt ein Tumorwachstum), (streßinduzierten) Geschwüren, ulceröser Colitis, Morbus Crohn, Herzinfarkten, allergischen Reaktionen auf Arzneimittel, Ischämie nach einer Wiederbelebung, oder Migränekopfschmerzen, oder in der Augenheilkunde, beispielsweise zur Behandlung diabetischer Retinopathie, altersbedingter Macularathrophie, Katarakten und Glaukom, lichtinduzierter Netzhautschädigung und in bei der Augenchirurgie verwendeten Spülungen, bei der Behandlung subarachnoidaler Blutungen und anschließendem Hirngefäßspasmus, globaler, cerebraler Ischämie, und bei der Verhinderung einer postischämischen Hirnschädigung, von Hirntumor (nervenschützend), Bell-Lähmung, oder sonstigen degenerativen neurologischen Störungen, Lebernekrose (beispielsweise aufgrund einer Virushepatitis), Strahlenschädigung (beispielsweise während einer Strahlenbehandlung oder aufgrund einer zufälligen Strahleneinwirkung), Herzschädigung nach Myocardischämie, einer Leibesfruchstrangulierung vor der Geburt und Leibesfruchthypoxiesyndrom, solcher Augenstörungen, wie Uveitis und Sehnervenentzündung und ischämischem Darm-Syndrom, Schädigung nach Herz-Lungen-Wiederbelebung, neurologischer oder cardiovaskulärer Chirurgie und aufgrund von Herzinfarkten, oder einer Augenschädigung nach augenchirurgischen Maßnahmen (beispielsweise Starchirurgie).

2. Tricyclisches Amin der Formel XXX gemäß der Definition in Anspruch 1, ausgenommen 6,7-Dimethoxy-2,4-di(1-piperidinyl)-9H-pyrimido[4,5-b]indol und 6,7-Dimethoxy-2,4-di(4-methyl-1-piperazinyl)-9H-pyrimido[4,5-b]indol.

3. Tricyclisches Amin nach Anspruch 2, wobei R₂₋₁ und R₂₋₂ zusammen mit dem Stickstoffatom, an dem sie hängen, 1-Pyrrolidinyl, 1-Piperazinyl, 1-Thiomorpholinyl oder 4-Methylpiperazin-1-yl bilden.

4. Tricyclisches Amin nach Anspruch 3, wobei R₂₋₁ und R₂₋₂ zusammen 1-Pyrrolidinyl und 1-Piperazinyl darstellen.

5. Tricyclisches Amin nach Anspruch 2, wobei R₄₋₁ und R₄₋₂ zusammen mit dem Stickstoffatom, an dem sie hängen, 1-Pyrrolidinyl, 1-Piperazinyl, 1-Thiomorpholinyl oder 4-Methylpiperazin-1-yl bilden.

6. Tricyclisches Amin nach Anspruch 5, wobei R₄₋₁ und R₄₋₂ zusammen 1-Pyrrolidinyl und 1-Piperazinyl darstellen.

7. Tricyclisches Amin nach Anspruch 2, wobei R₇ für -H, -CH₃, Phenyl, 2-(1-Morpholinyl)ethyl oder 2-(1-Piperazinyl)ethyl steht.

8. Tricyclisches Amin nach Anspruch 2 in Form eines Salzes mit einer Säure, ausgewählt aus Chlorwasserstoff-, Bromwasserstoff-, Methansulfon-, Schwefel-, Phosphor-, Salpeter-, Benzoe-, Citronen-, Wein-, Fumar- und Maleinsäure, CH₃-(CH₂)₀₋₄-COOH und HOOC-(CH₂)₀₋₄-COOH.

9. Tricyclisches Amin nach Anspruch 2, ausgewählt aus 5,6,7,8-Tetrahydro-9-methyl-2,4-di-(1-pyrrolidinyl)-5H-pyrimido[4,5-b]indol, 9-Methyl-2,4-di-(1-pyrrolidinyl)-9H-pyrimido[4,5-b]indol, 5,6,7,8-Tetrahydro-2,4-di(1-pyrrolidinyl)-1H-pyrimido[4,5-b]indol und 5,6,7,8-Tetrahydro-9-[2-(1-piperazinyl)ethyl]-2,4-di-(1-pyrrolidinyl)-5H-pyrimido[4,5-b]indol.

## Revendications

1. Utilisation d'un composé pour la production d'un médicament destiné à être utilisé dans le traitement/la prévention d'un traumatisme rachidien, d'une lésion cérébrale faible et/ou moyenne à grave, d'une hémorragie sous-arachnoïdienne et d'un ictus ischémique subséquent (thrombo-embolique), de l'asthme et de la réduction de la formation/sécrétion de mucus dans les poumons, de la dystrophie musculaire, de la toxicité cardiaque induite par l'adriamycine, de la maladie de Parkinson, de la maladie d'Alzheimer, d'autres troubles neurologiques dégénératifs, de la sclérose en plaques, d'une altération d'organes au cours d'une reperfusion après transplantation, d'un rejet de greffe cutanée, de chocs hémorragiques, traumatiques et septiques et d'états tels que des brûlures graves, le syndrome de détresse respiratoire de l'adulte (ARDS), de maladies inflammatoires telles que l'ostéoarthrite ou l'arthrite rhumatoïde, du syndrome néphrotique (immunologique), du lupus érythémateux disséminé, de réactions allergiques, du diabète, de l'athérosclérose, de l'inflammation (agents dermatologiques anti-inflammatoires et anti-psoriasiques), de l'emphysème, d'un cancer (métastase limite, croissance de tumeur limite), d'ulcères (induits par un stress), de la colite ulcérative, de la maladie de Crohn, des infarctus du myocarde, de réactions allergiques à des médicaments, de l'ischémie après réanimation ou des céphalées de la migraine, ou en ophtalmologie, par exemple dans le traitement de la rétinopathie diabétique, de la dégénérescence maculaire liée à l'âge, des cataractes et du glaucome, d'une altération rétinienne induite par la lumière, et dans des mélanges d'irrigation utilisés en chirurgie oculaire, dans le traitement de l'hémorragie sous-arachnoïdienne et du vasospasme cérébral subséquent, de l'ischémie cérébrale globale et dans la prévention d'une altération cérébrale post-ischémique, d'une tumeur cérébrale (effet neuro-protecteur), de la paralysie de Bell, d'autres troubles neurologiques dégénératifs, d'une nécrose hépatique (faisant par exemple suite à une hépatite virale), d'une altération par un rayonnement (par exemple au cours d'un traitement par irradiation ou par une exposition accidentelle à un rayonnement), d'une altération myocardique après une ischémie myocardique, de la strangulation du nourrisson avant la naissance et du syndrome hypoxique du nourrisson, de troubles ophtalmiques tels que l'uvéite et la névrite optique et du syndrome du colon ischémique, d'une altération après une réanimation cardiopulmonaire, une intervention chirurgicale neurologique ou cardio-vasculaire et un infarctus cardiaque, ou d'une altération oculaire après une intervention chirurgicale ophtalmique (par exemple une intervention chirurgicale pour le traitement de la cataracte), dans laquelle le composé est une amine hétérocyclique tricyclique de formule 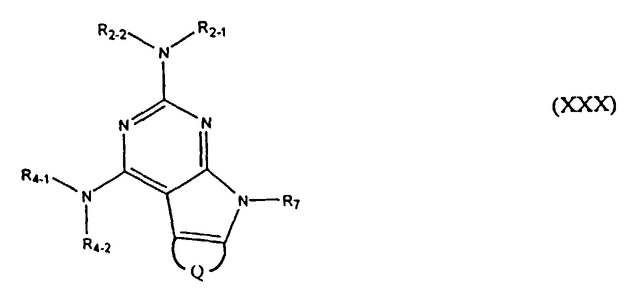 dans laquelle Q représente un groupe -(CH₂)₃₋₅ ou -CR₅₆₋₁ = CR₅₆₋₂ - CR₅₆₋₃ = CR₅₆₋₄ et dans laquelle R₅₆₋₁, R₅₆₋₂, R₅₆-₃ et R₅₆-₄ représentent des groupes -H, -F, -Cl, -Br, -OH, -OCH₃, -OCH₂-φ, -NO₂, alkyle en C₁ à C₃ , -NH₂, -NHCH₃, N(CH₃)₂ ou -CO₂R₅₆-₅ dans lequel R₅₆-₅ représente -H, un groupe alkyle en C₁ à C₄, aryle en C₆ à C₁₂ ou aralkyle en C₆ à C₁₂.
R₂₋₁ représente
(A) -H, ou
(B) un groupe alkyle en C₁ à C₈ facultativement substitué avec 1 à 4 groupes
(1) -F,
(2) -Cl,
(3) -OR₂₋₁₀ dans lequel R₂₋₁₀ représente un groupe
(a) -H
(b) alkyle en C₁ à C₄
(c) phosphate
(d) sulfate
(e) -Co-R₂₋₁₁ dans lequel R₂₋₁₁ représente un groupe alkyle en C₁ à C₄ ou aralkyle en C₆ à C₉,
(f) -CO-NR₂₋₁₂R₂₋₁₃ où R₂₋₁₂ et R₂₋₁₃ sont identiques ou différents et représentent -H ou des groupes alkyle en C₁ à C₃,
(g) sulfamate
(h) glucosyle
(i) galactosyle
(j) acide glucoronique
(k) maltosyle
(l) arabinosyle
(m) xylosyle
(n)-CO-CH(NH₂)-H,
(o) -CO-CH(NH₂)-CH₃,
(p) -CO-CH(NH₂)-CH(CH₃)₂,
(q) -CO-CH(NH₂)-CH₂CH(CH₃)₂,
(r) -CO-CH(NH₂)-CH(CH₃)-CH₂-CH₃,
(s) -CO-CH(NH₂)-CH₂-OH
(t) -CO-CH(NH₂)-CH(OH)-CH₃,
(u) -CO-CH(NH₂)-CH₂-φ,
(v) -CO-CH(NH₂)-CH₂-[*p*-phenyl]-OH,
(w) -CO-CH(NH₂)-CH₂-[2-indolyl]
(x) -CO-CH(NH₂)-CH₂-SH,
(y) -CO-CH(NH₂)-CH₂-CH₂-S-CH₃,
(z) -CO-C*H-NH-CH₂-CH₂-C*H₂
dans lequel le signe * indique que les atomes de carbone marqués sont liés l'un à l'autre pour former un noyau hétérocyclique,
(aa) -CO-C*H-NH-CH₂-CH(OH)-C*H₂ dans lequel le signe * indique que les atomes de carbone marqués sont liés l'un à l'autre pour former un noyau hétérocyclique,
(bb) -CO-CH(NH₂)-CH₂-COOH,
(cc) -CO-CH(NH₂)-CH₂-CONH₂,
(dd) -CO-CH(NH₂)-CH₂-CH₂-COOH,
(ee) -CO-CH(NH₂)-CH₂-CH₂-CONH₂,
(ff) -CO-CH(NH₂)-CH₂-C*-NH-CH=N-C*H=
dans lequel le signe * indique que les atomes de carbone marqués sont liés l'un à l'autre pour former un noyau hétérocyclique,
(gg) -CO-CH(NH₂)-CH₂-CH₂-CH₂-NH-C(=NH)-NH₂,
(hh) -CO-CH-(NH₂)-CH₂-CH₂-CH₂-CH₂-NH₂,
(ii) -CO-CH(NH₂)-CH₂-CH₂-CH(OH)-CH₂-NH₂,
(jj) -CO-CH₂-CH₂-NH₂,
(kk) -CO-CH₂-CH₂-CH₂-NH₂,
(ll) -CO-CH(NH₂)-CH₂-CH₂-CH₂-NH₂,
(mm) -CO-CH(NH₂)-CH₂-CH₂-CH₂-NH-CO-NH₂,
(nn) -CO-CH(NH₂)-CH₂-CH₂-OH, ou
(4) -N(R₂₋₁₄)₂ dans lequel les groupes R₂₋₁₄ peuvent être identiques ou différents et représentent un groupe
(a) alkyle en C₁ à C₆ facultativement substitué avec 1 à 3 groupes -OH ou -OCH₃,
(b) (alkyle en C₁ à C₆) carbonyle
(c) (alkoxy en C₁ à C₆) carbonyle
(d) (aryle en C₆ à C₁₂) alkyle
(e) φ
(f) -SO₂-(alkyle en C₁ à C₈)
(g) CH₃-C*-O-CO-O-C*-CH₂- dans lequel le signe * indique que les atomes de carbone marqués sont fixés par une double liaison pour former un noyau pentagonal, et
R₂₋₂ représente un groupe
(A) -H , ou
(B) alkyle en C₁ à C₈ facultativement substitué avec 1 à 4 groupes
(1) -F,
(2) -Cl,
(3) -OR₂₋₁₀ dans lequel R₂₋₁₀ répond à la définition précitée,
(4) -N(R₂₋₁₄)₂ dans lequel les groupes R₂₋₁₄ peuvent être identiques ou différents et répondent chacun à la définition précitée, ou
R₂₋₁ et R₂₋₂ sont pris conjointement avec l'atome d'azote auquel ils sont fixés pour former un noyau hétérocyclique choisi entre des noyaux
(A) 1-pyrrolidine facultativement substituée sur le carbone avec 1 à 3 groupes R₂₋₃, les groupes R₂₋₃ étant choisis entre des groupes
(1)alkyle en C₁ à C₆ facultativement substitué avec 1 à 3 substituants -OH ou -OCH₃
(2)alcényle en C₁ à C₆ facultativement substitué avec 1 à 3 substituants -OH ou -OCH₃
(3)(alkyle en C₁ à C₆) carbonyle
(4)(alkoxy en C₁ à C₆) carbonyle
(5)(aryle en C₆ à C₁₂) alkyle
(6) = O
(7) -OH
(8) -C≡N,
(9) -CO₂R₂₋₄ dans lequel R₂₋₄ représente un groupe
(a) -H
(b) alkyle en C₁ à C₄
(c) aryle en C₆ à C₁₂
(d) aralkyle en C₆ à C₁₂
(10) -NH₂
(11) -Cl
(12) -F
(13) -Br
(14) -φ facultativement substitué avec 1 à 3 substituants -F, -Cl, -Br, -OH, -OCH₃, -OCH₂-φ, -NO₂, alkyle en C₁ à C₃, -NH₂, -NHCH₃, N(CH₃)₂, -CO₂R₂₋₄ dans lequel R₂₋₄ répond à la définition précitée, ou
(15) -(CH₂)ₙ₄NR₂₋₆R₂₋₇ dans lequel R₂₋₆ et R₂₋₇ sont identiques ou différents et représentent des groupes alkyle en C₁ à C₄, ou bien peuvent être repris conjointement avec l'atome d'azote auquel ils sont fixés pour former le noyau hétérocyclique -N*-(CH₂)ₙ₅-R₂₋₈-(CH₂)ₙ₆*dans lequel le signe * indique que les atomes marqués sont liés l'un à l'autre avec pour résultat la formation d'un noyau, n₄ ayant une valeur de 0 à 3, n₅ ayant une valeur de 1 à 5, n₆ ayant une valeur de 0 à 3 et R₂₋₈ représentant un groupe
(a) -CH₂-,
(b) -O-,
(c) -S-, ou
(d) -NR₂₋₄ dans lequel R₂₋₄ répond à la définition précitée,
(B)1-pipéridinyle facultativement substitué sur le carbone avec 1 à 3 substituants R₂₋₃, les groupes R₂₋₃ répondant à la définition précitée
(C) 1-morpholinyle facultativement substitué sur le carbone avec 1 à 3 substituants R₂₋₃, les groupes R₂₋₃ répondant à la définition précitée
(D) 1-pipérazinyle facultativement substitué sur le carbone avec 1 à 3 substituants R₂₋₃, les groupes R₂₋₃ répondant à la définition précitée, et facultativement substitués en position 4 avec un groupe R₂₋₅, le groupe R₂₋₅ représentant un groupe
(1) alkyle en C₁ à C₆ facultativement substitué avec 1 à 3 substituants -OH ou -OCH₃
(2)(alkyle en C₁ à C₆) carbonyle
(3)(alkoxy en C₁ à C₆) carbonyle
(4)(aryle en C₆ à C₁₂) alkyle
(5) -φ
(6) -SO₂-(alkyle en C₁ à C₈)
(7) CH₃-C*-O-CO-O-C*-CH₂- dans lequel le signe * indique que les atomes de carbone marqués sont fixés à une double liaison pour former un noyau pentagonal,
(E) 1-aziridinyle facultativement substitué sur le carbone avec 1 ou 2 substituants R₂₋₃, les groupes R₂₋₃ répondant à la définition précitée,
(F) 1-azétidinyle facultativement substitué sur le carbone avec 1 à 3 substituants R₂₋₃, les groupes R₂₋₃ répondant à la définition précitée,
(G) 1-hexaméthylèneimino facultativement substitué sur le carbone avec 1 à 3 substituants à R₂₋₃, les groupes R₂₋₃ répondant à la définition précitée,
(H) 1-pyrrolyle facultativement substitué sur le carbone avec 1 à 3 substituants R₂₋₃, les groupes R R₂₋₃ répondant à la définition précitée,
(I) 1-imidazolyle facultativement substitué sur le carbone avec 1 à 3 substituants R₂₋₃, les groupes R₂₋₃ répondant à la définition précitée,
(J) 1-pyrazoyle facultativement substitué sur le carbone avec 1 à 3 substituants R₂₋₃, les groupes R₂₋₃ répondant à la définition précitée,
(K) 1-pyrazolidinyle facultativement substitué sur le carbone avec 1 à 3 substituants R₂₋₃, les groupes R₂₋₃ répondant à la définition précitée,
(L) 1, 2, 3-triazolyle facultativement substitué sur le carbone avec 1 à 3 substituants à R₂₋₃, les groupes R₂₋₃ répondant à la définition précitée,
(M) 1, 2, 4-triazolyle facultativement substitué sur le carbone avec 1 à 3 substituants R₂₋₃, les groupes R₂₋₃ répondant à la définition précitée
(N) 1-tétrazolyle facultativement substitué sur le carbone avec 1 à 3 substituants R₂₋₃, les groupes R₂₋₃ répondant à la définition précitée,
(O) 1-thiomorpholinyle facultativement substitué sur le carbone avec 1 à 3 substituants R₂₋₃, les groupes R₂₋₃ répondant à la définition précitée,
(P) 1-thiazolidinyle facultativement substitué sur le carbone avec 1 à 3 substituants R₂₋₃, les groupes R₂₋₃ répondant à la définition précitée, 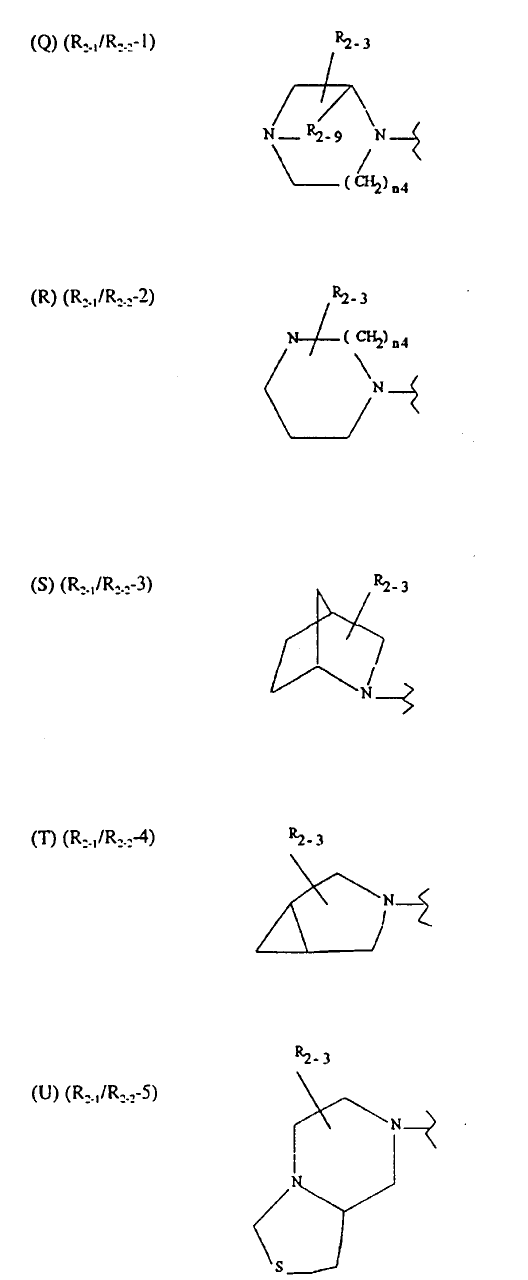 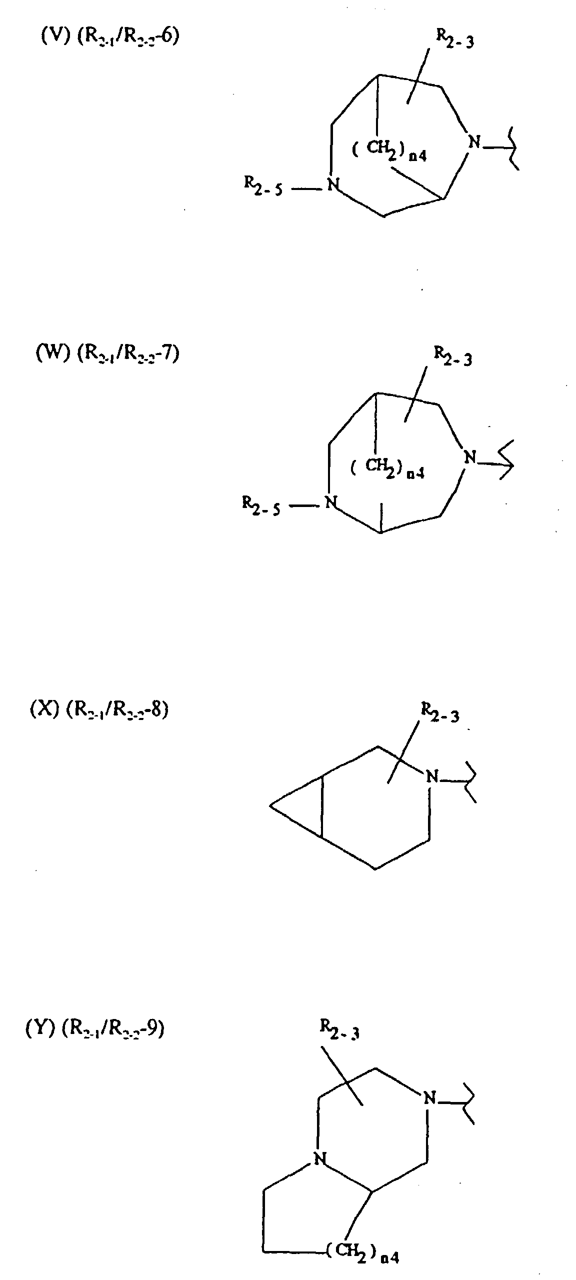 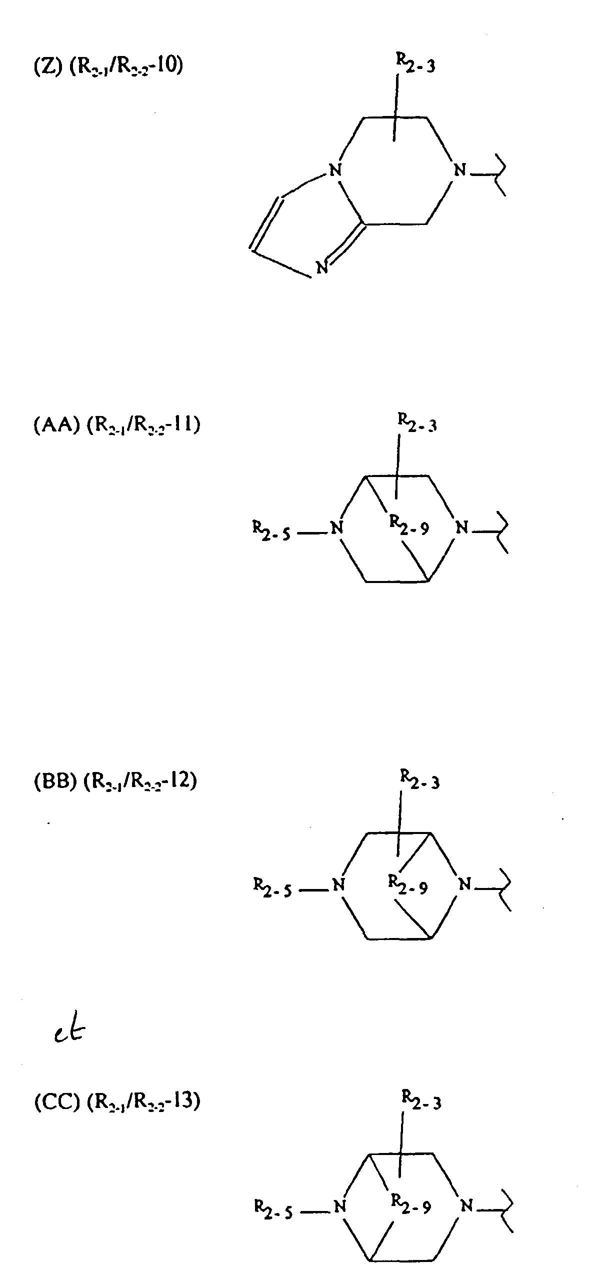 dans lesquels R₂₋₃ et R₂₋₅ répondent aux définitions précitées, et
R₂₋₉ représente un groupe
(A) -(CH₂)ₙ₄ dans lequel n₄ a une valeur de 1 à 3
(B) -CH₂OCH₂,
(C) -CH₂SCH₂,
(D) -CH₂SO₂CH₂,
(E) -CH₂S,
(F) -CH₂SO₂, ou
(G) -CH₂N(R₂₋₅)CH₂
dans lequel R₂₋₅ répond à la définition précitée ; sous réserve que R₂₋₁ et R₂₋₂ ne puissent représenter l'un et l'autre -H ;
R₄₋₁ répond à la définition mentionnée pour R₂₋₁, et peut être identique à ou différent de R₂₋₁,
R₄₋₂ répond à la définition mentionnée pour R₂₋₂, et peut être identique à ou différent de R₂₋₂, sous réserve que R₄₋₁ et R₄₋₂ ne puissent représenter l'un et l'autre -H ;
R7 représente un groupe
(A) -H
(B) alkyle en C₁ à C₈ facultativement substitué avec 1 à 4 substituants R₇₋₁, R₇₋₁ représentant un groupe
(1) -F, -Cl, -Br,
(2) alkyle en C₁ à C₄
(3) -CF₃
(4) -φ
(5) -OR₇₋₂ dans lequel R₇₋₂ représente un groupe
(a) -H
(b) alkyle en C₁ à C₄
(c) phosphate
(d) sulfate
(e) -CO-R₇₋₈ dans lequel R₇₋₈ représente un groupe alkyle en C₁ à C₄ ou aralkyle en C₆ à C₉
(f) -CO-NR₇₋₁₀R₇₋₁₁ dans lequel R₇₋₁₀ et R₇₋₁₁ sont identiques ou différents et représentent -H ou des groupes alkyle en C₁ à C₃,
(g) sulfamate
(h) glucosyle,
(i) galactosyle,
(j) acide glucuronique
(k) maltosyle
(l) arabinosyle
(m) xylosyle
(n) -CO-CH(NH₂)-H,
(o) -CO-CH(NH₂)-CH₃,
(p) -CO-CH(NH₂)-CH(CH₃)₂,
(q) -CO-CH(NH₂)-CH₂CH(CH₃)₂,
(r) -CO-CH(NH₂)-CH(CH₃)-CH₂-CH₃,
(s) -CO-CH(NH₂)-CH₂-OH
(t) -CO-CH(NH₂)-CH(OH)-CH₃,
(u) -CO-CH(NH₂)-CH₂-φ,
(v) -CO-CH(NH₂)-CH₂-[*p*-phenyl]-OH,
(w) -CO-CH(NH₂)-CH₂-[2-indolyl]
(x) -CO-CH(NH₂)-CH₂-SH,
(y) -CO-CH(NH₂)-CH₂-CH₂-S-CH₃,
(z) -CO-C*H-NH-CH₂-CH₂-C*H₂ dans lequel le signe * indique que les atomes de carbone marqués sont liés l'un a l'autre pour former un noyau hétérocyclique,
(aa) -CO-C*H-NH-CH₂-CH(OH)-C*H₂ dans lequel le signe * indique que les atomes de carbone marqués sont liés l'un à l'autre pour former un noyau hétérocyclique,
(bb) -CO-CH(NH₂)-CH₂-COOH,
(cc) -CO-CH(NH₂)-CH₂-CONH₂,
(dd) -CO-CH(NH₂)-CH₂-CH₂-COOH,
(ee) -CO-CH(NH₂)-CH₂-CH₂-CONH₂,
(ff) -CO-CH(NH₂)-CH₂-C⁻-NH-CH=N-CH⁻H=
dans lequel le signe * indique que les atomes de carbone marqués sont liés l'un à l'autre pour former un noyau hétérocyclique.
(gg)-CO-CH(NH₂)-CH₂-CH₂-CH₂-NH-C(=NH)-NH₂,
(hh)-CO-CH-(NH₂)-CH₂-CH₂-CH₂-CH₂-NH₂,
(ii)-CO-CH(NH₂)-CH₂-CH₂-CH(OH)-CH₂-NH₂,
(jj)-CO-CH₂-CH₂-NH₂,
(kk)-CO-CH₂-CH₂-CH₂-NH₂,
(ll)-CO-CH(NH₂)-CH₂-CH₂-CH₂-NH₂,
(mm)-CO-CH(NH₂)-CH₂-CH₂-CH₂-NH-CO-NH₂,
(nn)-CO-CH(NH₂)-CH₂-CH₂-OH,
(6) -SR₇₋₂ dans lequel R₇₋₂ répond à la définition précitée
(7) -NHR₇₋₃ dans lequel R₇₋₃ représente -H ou un groupe alkyle en C₁ à C₄
(8) -NR₇₋₄R₇₋₅ dans lequel R₇₋₄ et R₇₋₅ sont identiques ou différents et représentent des groupes alkyle en C₁ à C₄, ou bien peuvent être pris conjointement avec l'atome d'azote auxquels ils sont fixés pour former un groupe -N*-(CH₂)ₙ₁-R₅₋₆-(CH₂)ₙ₂* dans lequel le signe * indique que les atomes de carbone marqués sont liés l'un à l'autre avec pour résultat la formation d'un noyau, n₁ ayant une valeur de 1 à 5, n₂ ayant une valeur de 0 à 3 et R₅₋₆ représentant un groupe
(a) -CH₂-
(b) -O-
(c) -S-
(d) -NR₇₋₉ dans lequel R₇₋₉ représente un groupe
(i) alkyle en C₁ à C₆ facultativement substitué avec 1 à 3 substituants -OH ou -OCH₃,
(ii) (alkyle en C₁ à C₆) carbonyle
(iii) (alkoxy en C₁ à C₆) carbonyle
(iv) (aryle en C₆ à C₁₂) alkyle
(v) -φ
(vi) -SO₂-(alkyle en C₁ à C₈)
(vii) CH₃-C*-O-CO-O-C*-CH₂ dans lequel le signe * indique que les atomes de carbone marqués sont fixés par une double liaison pour former un noyau pentagonal,
(9) -(CH₂)ₙ₃CO₂R₇₋₂ dans lequel n₃ a une valeur de 0 à 6 et R₇₋₂ répond à la définition précitée,
(10) -(CH₂)ₙ₃CON(R₇₋₃)₂ dans lequel n₃ répond à la définition précitée et les groupes R₇₋₃ peuvent être identiques ou différents et répondent chacun à la définition précitée,
(11) -(CH₂)ₙ₃CONR₇₋₄R₇₋₅ dans lequel n₃, R₇₋₄ et R₇₋₅ répondent aux définitions précitées,
(12) -(CH₂)ₙ₁OR₇₋₂ dans lequel R₇₋₂ et n₁ répondent aux définitions précitées
(13) -(CH₂)ₙ₁OCOR₇₋₃ dans lequel R₇₋₃ et n₁ répondent aux définitions précitées,
(14) -(CH₂)ₙ₁SR₇₋₂ dans lequel R₇₋₂ et n₁ répondent aux définitions précitées,
(15) -(CH₂)ₙ₁NHR₇₋₃ dans lequel R₇₋₃ et n₁ répondent aux définitions précitées,
(16) -(CH₂)ₙ₁NR₇₋₄R₇₋₅ dans lequel R₇₋₄, R₇₋₅ et n₁ répondent aux définitions précitées
(C) -(CH₂)ₙ₃-φ, facultativement substitué avec 1 à 4 substituants R₇₋₁, R₇₋₁ et n₃ répondant aux définitions précitées,
(D) -(CH₂)n₃-pyridine -2, -3, ou -4-yle facultativement substitué avec 1 à 4 substituants R₇₋₁, n₃ et R₇₋₁ répondant aux définitions précitées,
(E) -(CH₂)ₙ₃-naphtaline -1 ou 2-yle facultativement substitué avec 1 à 4 substituants R₇₋₁, n₃ et R₇₋₁ répondant aux définitions précitées,
(F) -(CH₂)ₙ₃ CO₂R₇₋₂ dans lequel n₃, et R₇₋₂ répondent aux définitions précitées,
(G) -(CH₂)ₙ₃ CON(R₇₋₃)₂ dans lequel n₃ répond à la définition précitée et les groupes R₇₋₃ peuvent être identiques ou différents et répondent chacun à la définition précitée,
(H) -(CH₂)ₙ₃ CONR₇₋₄R₇₋₅ dans lequel n₃, R₇₋₄ et R₇₋₅ répondent aux définitions précitées,
(I) -(CH₂)ₙ₃SO₃R₇₋₂ dans lequel n₃ et R₇₋₂ répondent aux définitions précitées,
(J) cycloalkyle en C₃ à C₇ ; ou un de ses sels pharmaceutiquement acceptables.

2. Amine tricyclique de formule (XXX) répondant à la définition suivant la revendication 1, à l'exception du 6,7-diméthoxy-2,4-di(1-pipéridinyl)-9H-pyrimido [4,5-b] indole et du 6,7-diméthoxy-2,4-di(4-méthyl-1-pipérazinyl)-9H-pyrimido [4,5-b]indole.

3. Amine tricyclique suivant la revendication 2, dans laquelle R₂₋₁ et R₂₋₂ sont pris conjointement avec l'atome d'azote auquel ils sont fixés pour former un noyau 1-pyrrolidinyle, 1-pipérazinyle, 1-thiomorphonyle ou 4-méthylpipérazine-1-yle.

4. Amine tricyclique suivant la revendication 3, dans lequel R₂₋₁ et R₂₋₂, conjointement, représentent des noyaux 1-pyrrolidinyle et 1-pipérazinyle.

5. Amine tricyclique suivant la revendication 2, dans laquelle R₄₋₁ et R₄₋₂ sont pris conjointement avec l'atome d'azote auquel ils sont fixés pour former un noyau 1-pyrrolidinyle, 1-pipérazinyle, 1-thiomorpholinyle ou 4-méthylpipérazin-1-yle.

6. Amine tricyclique suivant la revendication 5, dans laquelle R₄₋₁ et R₄₋₂, conjointement, représentent des noyaux 1-pyrrolidinyle et 1-pipérazinyle.

7. Amine tricyclique suivant la revendication 2, dans laquelle R₇ représente -H, un groupe -CH3, phényle, 2-(1-morpholinyl)éthyle ou 2-(1-pipérazynyl)éthyle.

8. Amine tricyclique suivant la revendication 2, sous forme d'un sel, avec un acide choisi entre les acides chlorhydrique, bromhydrique, méthanesulfonique, sulfurique, phosphorique, nitrique, benzoïque, citrique, tartrique, fumarique et maléique, CH₃-(CH₂)₀₋₄-COOH et HOOC- (CH₂)₀₋₄-COOH.

9. Amine tricyclique suivant la revendication 2, choisi entre
le 5,6,7,8-tetrahydro-9-methyl-2,4-di-(1-pyrrolidinyl)-5H-pyrimido[4,5-b]indole,
le 9-methyl-2,4-di-(1-pyrrolidinyl)-9H-pyrimido[4,5-b]indole,
le 5,6,7,8-tetrahydro-2,4-di-(1-pyrrolidinyl)-1H-pyrimido[4,5-b]indole, et
le 5,6,7,8-tetrahydro-9-[2-(1-piperazinyl)ethyl]-2,4-di-(1-pyrrolidinyl)-5H-pyrimido[4,5-b]indole.
